# EUROPEAN PATENT SPECIFICATION

(11) **EP 3 199 181 B1**
(45) Date of publication and mention of the grant of the patent: **06.05.2020**
(21) Application number: 15844328.3
(22) Date of filing: 18.09.2015
(51) Int. Cl.: C12N 9/12, A61K 45/00, A61K 31/473, A61K 31/519, A61K 31/713, G01N 33/50

(54) **ANTI-INFLUENZA VIRUS AGENT, AND SCREENING METHOD FOR ANTI-INFLUENZA VIRUS AGENT**
WIRKSTOFF GEGEN INFLUENZAVIRUS UND SCREENING-VERFAHREN FÜR WIRKSTOFF GEGEN INFLUENZAVIRUS
AGENT CONTRE LE VIRUS DE LA GRIPPE ET PROCÉDÉ DE CRIBLAGE D'UN AGENT CONTRE LE VIRUS DE LA GRIPPE

(30) Priority: 22.09.2014 JP 2014192752
(43) Date of publication of application: 02.08.2017
(73) Proprietor: Japan Science and Technology Agency, Kawaguchi-shi, Saitama 332-0012 (JP)
(72) Inventor: KAWAOKA Yoshihiro, Tokyo 108-0071 (JP); WATANABE Tokiko, Tokyo 168-0082 (JP); KAWAKAMI Eiryo, Yokohama-shi Kanagawa 230-0051 (JP); WATANABE Shinji, Tokyo 168-0082 (JP)
(74) Representative: Plasseraud IP
(86) International application number: PCT/JP2015/076674
(87) International publication number: WO 2016/047592

(56) References cited:
- WO-A1-2011/098806
- WO-A2-2011/109217
- MEGAN L. SHAW: "The host interactome of influenza virus presents new potential targets for antiviral drugs : The host interactome of influenza virus", REVIEWS IN MEDICAL VIROLOGY, vol. 21, no. 6, 8 August 2011 (2011-08-08) , pages 358-369, XP055449027, GB ISSN: 1052-9276, DOI: 10.1002/rmv.703
- OLIVIA PERWITASARI ET AL: "Targeting Organic Anion Transporter 3 with Probenecid as a novel anti-influenza A virus strategy", ANTIMICROBIAL AGENTS AND CHEMOTHERAPY, vol. 57, no. 1, 5 November 2012 (2012-11-05), pages 475-483, XP055449022, ISSN: 0066-4804, DOI: 10.1128/AAC.01532-12
- Y.-J. SEO ET AL: "Sphingosine 1-Phosphate-Metabolizing Enzymes Control Influenza Virus Propagation and Viral Cytopathogenicity", JOURNAL OF VIROLOGY., vol. 84, no. 16, 15 August 2010 (2010-08-15), pages 8124-8131, XP055420202, US ISSN: 0022-538X, DOI: 10.1128/JVI.00510-10
- Claire E Stewart ET AL: "Inhibitors of the interferon response enhance virus replication in vitro", PloS one, 12 November 2014 (2014-11-12), page e112014, XP055449236, United States DOI: 10.1371/journal.pone.0112014 Retrieved from the Internet: URL:http://journals.plos.org/plosone/artic le/file?id=10.1371/journal.pone.0112014&ty pe=printable [retrieved on 2018-02-08]
- WATANABE TOKIKO ET AL: "Influenza Virus-Host Interactome Screen as a Platform for Antiviral Drug Development", CELL HOST & MICROBE, ELSEVIER, NL, vol. 16, no. 6, 20 November 2014 (2014-11-20), pages 795-805, XP029112969, ISSN: 1931-3128, DOI: 10.1016/J.CHOM.2014.11.002
- SEO ET AL.: 'Sphingosine 1-Phosphate- Metabolizing Enzymes Control Influenza Virus Propagation and Viral Cytopathogenicity' JOURNAL OF VIROLOGY vol. 84, no. 16, 2010, pages 8124 - 8131, XP055420202
- WATANABE ET AL.: 'Influenza virus-host interactome screen as a platform for antiviral drug development' CELL HOST & MICROBE vol. 16, no. 6, 10 December 2014, pages 795 - 805, XP029112969

## Description

### [Technical Field]

The present invention relates to an anti-influenza virus agent that targets biomolecules in host cells including human cells and a method of screening candidate molecules for the anti-influenza virus agent.

### [Background Art]

Influenza viruses cause epidemic diseases every year and sometimes cause pandemic diseases taking millions of victims. Therefore, the development of more effective anti-influenza virus agents is necessary. As target molecules of anti-influenza virus agents, biomolecules of host cells that contribute to infection and replication of viruses are more preferable than virus proteins. This is because biomolecules of host cells are less prone to mutation due to drug selective pressure than virus proteins.

In recent years, by 6 types of genome-wide screening, a total of 1449 human genes (including human orthologs of 110 fly (Drosophila) genes) that are considered to play some role in the influenza virus life cycle have been identified (refer to Non Patent Literatures 1 to 6). Although genome-wide screening results only partially match, the reason for this is speculated to be related to the different experimental conditions.

### [Citation List]

### [Non Patent Literature]

[Non Patent Literature 1]
   Brass, et al., Cell, 2009, vol. 139, p. 1243 to 1254.
[Non Patent Literature 2]
   Hao, et al., Nature, 2008, vol. 454, p. 890 to 893.
[Non Patent Literature 3]
   Karlas, et al., Nature, 2010, vol.463, p.818 to 822.
[Non Patent Literature 4]
   Konig, et al., Nature, 2010, vol. 463, p. 813 to 817.
[Non Patent Literature 5]
   Shapira, et al., Cell, 2009, vol. 139, p. 1255 to 1267.
[Non Patent Literature 6]
   Sui, et al., Virology, 2009, vol. 387, p. 473 to 481.
[Non Patent Literature 7]
   Neumann, et al., Proceedings of the National Academy of Sciences of the United States of America, 1999, vol. 96, p. 9345 to 9350.
[Non Patent Literature 8]
   Tobita, et al., Medical microbiology and immunology, 1975, vol. 162, p. 9 to 14.
[Non Patent Literature 9]
   Ozawa et al., Journal of General Virology, 2011, vol. 92, p. 2879 to 2888.
[Non Patent Literature 10]
   Octaviani et al., Journal of Virology, 2010, vol. 84, p. 10918 to 10922.
[Non Patent Literature 11]
   Kawakami et al., Journal of Virological Methods, 2011, vol. 173, p. 1 to 6.
[Non Patent Literature 12]
   Wishart et al., Nucleic Acids Research, 2006, vol. 34, p. D668 to 672.
[Non Patent Literature 13]
   Patterson et al., Journal of General Virology, 1979, vol .43, p. 223 to 229.
[Non Patent Literature 14]
   Widjaja et al., Journal of Virology, 2010, vol. 84, p. 9625 to 9631.
[Non Patent Literature 15]
   Noda et al., Nature, 2006, vol. 439, p. 490 to 492.
[Non Patent Literature 16]
   Shaw ML, Reviews in medical virology, 2011, vol. 21,6, p. 358 to 369.
   This document discloses that the host interactome of Influenza virus presents new potential targets for antiviral drugs.
[Non Patent Literature 17]
   Perwitasari et al., Antimicrob. agents and chemoth., 2013, vol. 57, p. 475 to 483.
   This document discloses targeting organic anion transporter 3 with probenecid as a novel anti-influenza a virus strategy.
[Non Patent Literature 18]
   Y-J Seo et al., Journal of virology, 2010, vol. 84 p. 8124 to 8131.
   This document discloses that sphingosine 1-phosphate-metabolizing enzymes control influenza virus propagation and viral cytopathogenicity.
[Non Patent Literature 19]
   Stewart, CE et al., PloS one, 12 Nov. 2014, vol. 9,11
   This document discloses that inhibitors of the interferon response enhance virus replication in vitro.
[Non Patent Literature 20]
   Watanabe et al. Cell host & microbe, 2014, vol. 16, p. 795 to 805.
   This document discloses Influenza virus-host interactome screen as a platform for antiviral drug development.

### [Summary of Invention]

### [Technical Problem]

According to several types of genome-wide screening, proteins in host cells related to influenza virus replication and the like have been identified. However, a mechanism by which such proteins influence influenza infection has not been completely clarified, and a possibility of such proteins being candidate molecules for a novel anti-influenza virus agent has not been clarified.

The present invention provides an anti-influenza virus agent that targets JAK1 protein involved in an influenza virus life cycle that is a biomolecule within a host cell such as a human cell and a screening method for a candidate molecule for a novel anti-influenza virus agent.

### [Solution to Problem]

The inventors have conducted extensive studies, identified 1292 human proteins that interact with influenza virus proteins according to an immunoprecipitation method using a cell lysate of HEK293 cells derived from a human embryonic kidney, and then, among these human proteins, identified proteins in which influenza virus replication was significantly suppressed without significantly impairing a proliferative ability of host cells when an expression level was suppressed by using RNA interference, and thus completed the present invention.

The invention is defined by the claims. Any subject-matter falling outside the scope of the claims is provided for information purposes only.

### [Advantageous Effects of Invention]

Since an anti-influenza virus agent according to the present invention targets a protein in a host cell rather than a substance of an influenza virus, it is advantageous in that mutation due to drug selective pressure is less likely to occur.

In addition, according to a screening method for an anti-influenza virus agent according to the present invention, it is possible to screen a candidate molecule for an anti-influenza virus agent targeting a protein of a host cell involved in influenza virus infection and replication. Therefore, the method is suitable for designing and preparing a novel anti-influenza virus agent.

### [Brief Description of Drawings]

Fig. 1 shows diagrams of measurement results of virus titers (log₁₀(PFU/mL)) and cell viability (%) of cells treated with Golgicide A in Example 2.
Fig. 2 shows diagrams of measurement results of virus titers (log₁₀/PFU/mL)) and cell viability (%) of cells treated with Ruxolitinib in Example 2.
Fig. 3 shows electron microscope images of cells into which a control siRNA is introduced (upper side) and cells into which siRNA of JAK1 gene is introduced (lower side) in Example 3.

### Detailed description

### <Anti-influenza virus agent>

An anti-influenza virus agent according to the present invention is has an effect of suppressing expression of a JAK1 gene (hereinafter referred to as an "anti-Flu gene" in some cases) encoding a host cell protein that interacts with an influenza virus protein and that, when expression in a host cell is suppressed, suppresses influenza virus replication without excessively impairing a proliferative ability of the host cell, or an effect of suppressing a function of the protein. Specific examples of the anti-Flu gene may include CHERP gene, DDX21 gene, DNAJC11 gene, EEF1A2 gene, HNRNPK gene, ITM2B gene, MRCL3 gene, MYH10 gene, NDUFS8 gene, PSMD13 gene, RPL26 gene, SDF2L1 gene, SDF4 gene, SFRS2B gene, SNRPC gene, SQSTM1 gene, TAF15 gene, TOMM40 gene, TRM2B gene, USP9X gene, BASP1 gene, THOC2 gene, PPP6C gene, TESC gene, PCDHB12 gene, CCDC56 gene, CLTC gene, CYC1 gene, NIBP gene, ZC3H15 gene, C14orf173 gene, ANP32B gene, BAG3 gene, BRD8 gene, CCDC135 gene, DDX55 gene, DPM3 gene, EEF2 gene, IGF2BP2 gene, KRT14 gene, S100A4 gene, ASCC3L1 gene, C19orf43 gene, DKFZp564K142 gene, FAM73B gene, FLJ20303 gene, GBF1 gene, NCLN gene, LRPPRC gene, and RCN1 gene. As will be shown in the following examples, a protein that the anti-Flu gene encodes is a protein that directly or indirectly binds to 11 types of influenza virus proteins (PB2, PB1, PA, HA, NP, NA, M1, M2, NS1, NS2, and PB1-F2) and an interaction between them plays an important role in the influenza virus life cycle.

Among anti-Flu genes, JAK1 gene, CHERP gene, DDX21 gene, DNAJC11 gene, EEF1A2 gene, HNRNPK gene, ITM2B gene, MRCL3 gene, MYH10 gene, NDUFS8 gene, PSMD13 gene, RPL26 gene, SDF2L1 gene, SDF4 gene, SFRS2B gene, SNRPC gene, SQSTM1 gene, TAF15 gene, TOMM40 gene, TRM2B gene, USP9X gene, BASP1 gene, THOC2 gene, PPP6C gene, TESC gene, and PCDHB12 gene are genes that encode proteins involved in incorporation of the influenza virus vRNA or NP protein in host cells into influenza virus-like particles. Therefore, when a substance having an effect of suppressing expression of such genes or an effect of suppressing a function of a protein that the gene encodes is introduced into host cells, incorporation of the vRNA or NP protein into influenza virus-like particles in host cells is suppressed. As a result, an anti-influenza virus effect (an influenza virus replication inhibitory effect) is obtained.

Among anti-Flu genes, CCDC56 gene, CLTC gene, CYC1 gene, NIBP gene, ZC3H15 gene, C14orf173 gene, ANP32B gene, BAG3 gene, BRD8 gene, CCDC135 gene, DDX55 gene, DPM3 gene, EEF2 gene, IGF2BP2 gene, KRT14 gene, and S100A4 gene encode proteins involved in influenza virus replication or transcription in host cells. Therefore, when a substance having an effect of suppressing expression of such genes or an effect of suppressing a function of a protein that the gene encodes is introduced into host cells, influenza virus replication or transcription in the host cells is suppressed. As a result, an anti-influenza virus effect is obtained.

Among anti-Flu genes, ASCC3L1 gene, BRD8 gene, C19orf43 gene, DDX55 gene, DKFZp564K142 gene, DPM3 gene, EEF2 gene, FAM73B gene, FLJ20303 gene, GBF1 gene, NCLN gene, C14orf173 gene, LRPPRC gene, and RCN1 gene encode proteins involved in the formation of influenza virus-like particles in host cells. Therefore, when a substance having an effect of suppressing expression of such genes or an effect of suppressing a function of a protein that the gene encodes is introduced into host cells, the formation of influenza virus-like particles in the host cells is suppressed. As a result, an anti-influenza virus effect is obtained

As the anti-influenza virus agent according to the present invention, an agent including a substance having an effect of suppressing expression of anti-Flu genes according to the present invention as an active ingredient may be exemplified. As a substance having an effect of suppressing expression of the anti-Flu gene, for example, a small interfering RNA (siRNA), a short hairpin RNA (shRNA) or a micro RNA (miRNA) having a double-stranded structure including a sense strand and an antisense strand of a partial region (an RNA interference (RNAi) target region) of cDNA of the anti-Flu gene may be exemplified. In addition, an RNAi inducible vector through which siRNA and the like can be produced in host cells may be used. siRNA, shRNA, miRNA, and an RNAi inducible vector can be designed and prepared from base sequence information of cDNA of a target anti-Flu gene using a general method. In addition, the RNAi inducible vector can be prepared by inserting a base sequence of an RNAi target region into a base sequence of various commercially available RNAi vectors.

As the anti-influenza virus agent according to the present invention, an agent including a substance having an effect of suppressing a function (hereinafter simply referred to as a "function of the anti-Flu gene according to the present invention") of a protein encoded by the anti-Flu gene according to the present invention as an active ingredient may be exemplified. As the substance having an effect of suppressing the function of the anti-Flu gene, like an antibody for a protein (hereinafter simply referred to as an "anti-Flu protein according to the present invention") that an anti-Flu gene according to the present invention encodes, a substance binding to the anti-Flu protein according to the present invention and suppressing a function of the protein may be exemplified. The antibody may be a monoclonal antibody or a polyclonal antibody. In addition, the antibody may be an artificially synthesized antibody such as a chimeric antibody, a single chain antibody, and a humanized antibody. Such antibodies can be prepared using a general method.

When the anti-Flu protein according to the present invention is an enzyme, as a substance having an effect of suppressing the function of the anti-Flu gene, an inhibitor for the enzyme may be used.

As the anti-influenza virus agent according to the present invention, a substance having an effect of suppressing expression or function of one type of anti-Flu gene or a substance having an effect of suppressing expression or functions of two or more types of anti-Flu genes may be used.

As the anti-influenza virus agent, a substance having an effect of suppressing expression or function of at least one anti-Flu gene selected from the group including JAK1 gene, GBF1 gene, and USP9X gene is preferable, a substance having an effect of suppressing expression or function of at least one anti-Flu gene selected from the group including JAK1 gene and GBF1 gene is more preferable, and a substance having an effect of suppressing expression or function of JAK1 gene is most preferable.

As a substance having an effect of suppressing a function of JAK1 gene, JAK inhibitors such as Ruxolitinib (CAS No.: 941678-49-5) and Tofacitinib (CAS No.: 477600-75-2) may be exemplified. Ruxolitinib approved as a therapeutic agent for myelofibrosis and Tofacitinib approved as an anti-rheumatic agent are substances that can be used on the human body relatively safely. In addition, as a substance having an effect of suppressing a function of GBF1 gene, Golgicide A (CAS No.: 1005036-73-6) may be exemplified. Golgicide A can suppress influenza virus replication without influencing proliferation of host cells when a dose concentration is appropriately set.

In addition, JAK inhibitors such as Tofacitinib (CP-690550) Citrate
(3-((3R,4R)-4-methyl-3-(methyl(7H-pyrrolo[2,3-d]pyrimidin-4-yl)amino)piperidin-1-yl)-3-oxopropanenitrile), Tyrphostin B42 (AG-490)
((E)-N-benzyl-2-cyano-3-(3,4-dihydroxyphenyl)acrylamide), Baricitinib (LY3009104, INCB028050)
(2-[1-ethylsulfonyl-3-[4-(7H-pyrrolo[2,3-d]pyrimidin-4-yl)pyrazol-1-yl]azetidin-3-yl]ace tonitrile), AT9283
(1-cyclopropyl-3-(3-(5-(morpholinomethyl)-1H-benzo[d]imidazol-2-yl)-1H-pyrazol-4-yl )urea), Momelotinib (CYT387)
(N-(cyanomethyl)-4-(2-(4-morpholinophenylamino)pyrimidin-4-yl)benzamide), CEP33779 ([1,2,4]triazolo[1,5-a]pyridin-2-amine,
N-[3-(4-methyl-1-piperazinyl)phenyl]-8-[4-(methylsulfonyl)phenyl]-), NVP-BSK805 (8-(3,5-difluoro-4-(morpholinomethyl)phenyl)-2-(1-(piperidin-4-yl)-1H-pyrazol-4-yl)qui noxaline), ZM39923
(1-propanone, 3-[(1-methylethyl)(phenylmethyl)amino]-1-(2-naphthalenyl)-, hydrochloride (1:1)), Filgotinib (GLPG0634)
(N-[5-[4-[(1,1-dioxido-4-thiomorpholinyl)methyl]phenyl][1,2,4]triazolo[1,5-a]pyridin-2-yl]cyclopropanecarboxamide), JANEX-1
(4- [(6,7-dimethoxy-4-quinazolinyl)amino] -phenol), NVP-BSK805 (4-[[2, 6-difluoro-4-[3-(1-piperidin-4-ylpyrazol-4-yl)quinoxalin-5-yl]phenyl]methyl]morpholine ; dihydrochloride), and SB1317 (20-oxa-5,7,14,27-tetraazatetracyclo[19.3.1.12,6.18, 12]heptacosa-1(25),2,4,6(27),8,10,12(26),16,21,23-decaene,14-methyl-) may be used as the anti-influenza virus agent. In addition, as a substance having an effect of suppressing a function of USP9X gene, WP1130 (Degrasyn)
((2E)-3-(6-bromo-2-pyridinyl)-2-cyano-N-[1S-phenylbutyl]-2-propenamide) which is a DUB inhibitor may be exemplified.

When the substance having an effect of suppressing expression of anti-Flu genes according to the present invention is used as an active ingredient of the anti-influenza virus agent according to the present invention, an expression level of anti-Flu genes is preferably reduced by 50% or more, more preferably reduced by 75% or more, and most preferably reduced by 80% or more with respect to a case in which the anti-influenza virus agent is absent. Similarly, when the substance having an effect of suppressing the function of the anti-Flu gene is used as an active ingredient of the anti-influenza virus agent according to the present invention, the function of the anti-Flu gene is preferably degraded by 50% or more, more preferably degraded by 75% or more, and most preferably degraded by 80% or more with respect to a case in which the anti-influenza virus agent is absent.

The anti-influenza virus agent according to the present invention can be used for preventing influenza virus infection in animals and treating animals infected with an influenza virus. As animals into which the anti-influenza virus agent according to the present invention is introduced to prevent influenza virus infection or treat influenza, mammals such as humans, monkeys, pigs, horses, dogs, cats, and tigers, and birds such as chickens, ducks, quails, geese, ducks, turkeys, budgerigars, parrots, mandarin ducks, and swans may be exemplified. As the anti-influenza virus agent according to the present invention, a substance for preventing influenza virus infection in humans and treating animals infected with an influenza virus is preferable.

An influenza treatment is performed by administering an effective amount of the anti-influenza virus agent according to the present invention to animals infected with an influenza virus or animals in which prevention of an influenza virus infection is needed. An effective amount of the anti-influenza virus agent may be an amount at which an amount of influenza viruses is reduced in animals to which the agent is administered than animals to which the agent is not administered, or an amount at which influenza virus infection can be prevented. In addition, an effective amount of the anti-influenza virus agent is preferably an amount at which no serious side effects are caused by the anti-influenza virus agent. An effective amount of the anti-influenza virus agents can be calculated experimentally in consideration of a type of the anti-influenza virus agent, a type of an administration target animal, an administration method and the like. For example, when the agent is administered to a laboratory animal infected with an influenza virus, an amount at which an amount of influenza viruses in the body of the laboratory animal can be 70% or less, preferably 80% or less and more preferably 90% or less in PFU with respect to a laboratory animal to which the agent is not administered can be defined as an effective amount.

The anti-influenza virus agent according to the present invention can be formulated into dosage forms suitable for various administration forms such as oral administration, intravenous injection, direct administration into a nasal cavity or a buccal cavity, and transdermal administration using a general method. As the dosage form, a tablet, a powder, granules, a capsule, a chewable tablet, a syrup, a solution, a suspension, an injectable solution, a gargle, a spray, a patch, and an ointment may be exemplified.

The anti-influenza virus agent according to the present invention may include various additives in addition to the substance having an effect of suppressing expression or function of the anti-Flu gene. As the additive, an excipient, a binder, a lubricant, a wetting agent, a solvent, a disintegrant, a solubilizing agent, a suspending agent, an emulsifier, an isotonizing agent, a stabilizer, a buffering agent, a preservative agent, an antioxidant agent, a flavoring agent, and a colorant may be exemplified. Among additives that are pharmaceutically acceptable substances and used for pharmaceutical formulation, an appropriate additive can be selected and used.

### <Screening method for an anti-influenza virus agent>

A screening method for an anti-influenza virus agent according to the present invention (hereinafter referred to as a "screening method according to the present invention" in some cases) is a method of screening a candidate compound for an anti-influenza virus agent. The method includes screening a candidate compound capable of suppressing or inhibiting expression or the function of the anti-Flu gene according to the present invention as a candidate compound for the anti-influenza virus agent. The screening method according to the present invention may be a method of screening a substance capable of suppressing or inhibiting expression or a function of one type of anti-Flu genes or a method of screening a substance capable of suppressing or inhibiting expression or functions of two or more types of anti-Flu genes.

Specifically, screening of a substance capable of suppressing or inhibiting expression of anti-Flu genes is performed such that a target compound to be evaluated as a candidate compound for the anti-influenza virus agent is first introduced into cells and it is examined whether expression of anti-Flu genes is suppressed. When expression of anti-Flu genes is significantly suppressed, the compound is selected as the candidate compound for the anti-influenza virus agent. That is, a process in which a target compound to be evaluated as a candidate compound for the anti-influenza virus agent is introduced into cells, a process in which an expression level of anti-Flu genes in the cells into which the compound is introduced is measured, and a process in which, when an expression level of the anti-Flu genes is significantly lower than an expression level of the anti-Flu genes in cells into which the compound is not yet introduced, the compound is selected as the candidate compound for the anti-influenza virus agent are performed.

Cells used in screening are preferably cells of organism species serving as hosts of an influenza virus. In consideration of the convenience of handling, cultured cell lines of mammals and birds are more preferable, and cultured cell lines of a human are most preferable. In addition, the evaluation target compound can be introduced into cells using an electroporation method, a lipofection method, a calcium phosphate method and the like. When the evaluation target compound is a low molecular compound, the compound is added to a culture solution, and thus the compound can be introduced into cells.

A change in the expression level of anti-Flu genes may be evaluated at the level of mRNA or may be evaluated at the level of protein. For example, it is possible to quantitatively compare an expression level of anti-Flu genes by using a nucleic acid amplification reaction of an RT-PCR method and the like or through an immunohistochemical method or Western blotting. Specifically, for example, PCR in which the full length or a part of cDNA of anti-Flu genes is amplified by using cDNA as a template synthesized by a reverse transcription reaction from RNA extracted from cells cultured for 1 to 2 days while the evaluation target compound is introduced is performed. When an amount of the obtained amplified product is significantly lower than an amount of an amplified product obtained in the same manner from cells into which the compound is not yet introduced, it is evaluated that the compound can suppress or inhibit expression of anti-Flu genes. In addition, for example, anti-Flu proteins in cells cultured for 1 to 2 days while the evaluation target compound is introduced and in cells into which the compound is not yet introduced, iare stained using fluorescent-labeled antibodies, and fluorescence intensities of each cell are compared. When a fluorescence intensity per cell of cells into which the compound is introduced is significantly lower than that of cells into which the compound is not introduced, it is evaluated that the compound is capable of suppressing or inhibiting expression of anti-Flu genes. In addition, for example, cell extract liquids (lysates) of cells cultured for 1 to 2 days while the evaluation target compound is introduced and of cells into which the compound is not yet introduced are subjected to electrophoresis, separated, and then transcribed to membranes. Anti-Flu protein bands on the membranes are stained using fluorescent-labeled antibodies and fluorescence intensities of the bands are compared. When a fluorescence intensity of the anti-Flu protein band derived from the cells into which the compound is introduced is significantly lower than that derived from the cells into which the compound is not introduced, it is evaluated that the compound is capable of suppressing or inhibiting expression of anti-Flu genes.

When a function of the anti-Flu protein is an interaction with a specific biomolecule, for example, when a binding assay of the anti-Flu protein and the specific biomolecule is performed in the presence and absence of the evaluation target compound, and a connectivity between the anti-Flu protein and the specific biomolecule in the presence of the evaluation target compound is lower than that in the absence of the evaluation target compound, it is evaluated that the compound is capable of suppressing or inhibiting the function of the anti-Flu gene. In addition, when the anti-Flu protein is an enzyme, for example, when enzyme activity of the anti-Flu protein is measured in the presence and absence of the evaluation target compound and the enzyme activity of the anti-Flu protein in the presence of the evaluation target compound is lower than that in the absence of the evaluation target compound, it is evaluated that the compound is capable of suppressing or inhibiting the function of the anti-Flu gene.

### [Examples]

Next, the present invention will be described in further detail with reference to examples but the present invention is not limited to the following examples.

### <Cell culture>

In the following examples, HEK293 cells and A549 cells (derived from human lung epithelial cells) were cultured in a DMEM medium (commercially available from Sigma Aldrich) containing 10% fetal calf serum (FCS) and antibiotics under a 5% carbon dioxide atmosphere at 37°C. Madin-Darby canine kidney (MDCK) cells were cultured in a 5% newborn calf serum (NCS)-containing Eagle's minimum essential medium (Eagle's MEM) (commercially available from GIBCO) under a 5% carbon dioxide atmosphere at 37°C.

### <Influenza viruses>

Influenza viruses used in the following examples were A type influenza viruses (A/WSN/33, H1N1 subtype; WSN) unless otherwise described. The viruses were human-derived influenza viruses adapted to mice, prepared by a method (refer to Non Patent Literature 7) using reverse genetics, and propagated in MDCK cells. In addition, virus titers were measured by a plaque assay using MDCK cells (refer to Non Patent Literature 8).

PB2-KO/Rluc viruses (PB2 knockout viruses possessing a firefly luciferase gene) were replication-incompetent viruses and include a reporter gene encoding Renilla luciferase in a coding region of polymerase PB2 protein. PB2-KO/Rluc viruses were generated with pPolIPB2(120)Rluc(120) (a plasmid encoding Renilla luciferase flanked by 120 120 N- and C-terminal nucleotides derived from the PB2 segment) and a plasmid encoding the remaining seven viral RNA segments. PB2-KO/Rluc viruses were propagated and titers thereof were measured in MDCK cells stably expressing the PB2 protein (refer to Non Patent Literature 9).

### <Antibodies>

Mouse anti-HA antibody (WS3-54), mouse anti-NA antibody (WS5-29), and mouse anti-M1 antibody (WS-27/52) used in the following example provided from National Institute of Infectious Diseases (chief researcher Emi Takashita) were used. Mouse anti-Aichi NP antibody (2S-347/3) and rabbit anti-WSN virus antibody (R309) prepared by the inventors using a general method were used. Anti β-actin (AC-74) antibody purchased from Sigma Aldrich were used.

### [Example 1]

### <Identification of host proteins that interact with influenza virus proteins>

First, the inventors identified host proteins that interacted with influenza virus proteins using an immunoprecipitation method.

Specifically, first, a plasmid encoding a Flag fusion protein in which Flag tag was fused to the N-terminus or the C-terminus for 11 types of influenza virus proteins (PB2, PB1, PA, HA, NP, NA, M1, M2, NS1, NS2, and PB1-F2) was transfected into HEK293 cells, respectively. Transfection was performed using a TransIT 293 reagent (commercially available from Mirus Bio Corp.). Cells cultured for 24 hours after the transfection were mixed in a cell lysis buffer (50 mM Tris-HCl (pH 7.5), 150 mM NaCl, 1 mM EDTA, 0.5% Nonidet P-40, protease inhibitor mixture Complete Mini (Roche, Mannheim, Germany)), incubated at 4°C for 1 hour, and lysed to obtain a lysate. A supernatant collected by centrifuging the obtained lysate was added to an affinity gel (anti-FLAG M2 Affinity Gel commercially available from Sigma Aldrich) to which anti-Flag antibodies were bound and incubated at 4°C for 18 hours. Then, the affinity gel was washed three times with cell lysis buffer, then washed twice with an immunoprecipitation (IP) buffer (50 mM Tris-HCl (pH 7.5), 150 mM NaCl, 1 mM EDTA), and was stirred with an IP buffer containing 0.5 mg/mL of FLAG peptides (F1804, commercially available from Sigma Aldrich) at 4 °C for 2 hours. Then, the affinity gel was removed through centrifugation and a supernatant was collected. The collected supernatant was filtrated through an Ultrafree-MC filter (commercially available from Merck Millipore Corporation), and then the contained protein was identified through nano LC-MS/MS (nanoflow liquid chromatography tandem mass spectrometry) analysis. Q-STAR Elite (commercially available from AB SCIEX) coupled with Dina (commercially available from KYA technologies corporation) was used to analyze the mass spectrometry data. Co-immunoprecipitated proteins of HEK293 cells (host cells) were identified by comparing the obtained MS/MS signal with RefSeq (human protein database of National Center for Biotechnology Information). For this comparison, Mascot algorithm (version 2.2.04; commercially available from Matrix Science) was used under the following conditions: variable modifications, oxidation (Met), N-acetylation; maximum missed cleavages, 2; peptide mass tolerance, 200 ppm; MS/MS tolerance, 0.5 Da.). Protein identification required at least one MS/MS signal with a Mascot score that exceeded significantly the threshold value.

As a result, 388 host proteins were co-immunoprecipitated with PB2 proteins, 322 host proteins were co-immunoprecipitated with PB1 proteins, 304 host proteins were co-immunoprecipitated with PA proteins, 351 host proteins were co-immunoprecipitated with HA proteins, 574 host proteins were co-immunoprecipitated with NP proteins, 675 host proteins were co-immunoprecipitated with NA proteins, 659 host proteins were co-immunoprecipitated with M1 proteins, 531 host proteins were co-immunoprecipitated with M2 proteins, 113 host proteins were co-immunoprecipitated with NS1 proteins, 42 host proteins were co-immunoprecipitated with NS2 proteins, and 81 host proteins were co-immunoprecipitated with PB1-F2 proteins. That is, a total of 1292 host proteins were co-immunoprecipitated with any of 11 types of influenza virus proteins.

### <siRNA>

Next, RNA interference was performed on genes that encoded the 1292 host proteins identified by immunoprecipitation and it was examined whether these proteins were actually involved in influenza virus replication. 2 types of siRNA were selected from genome-wide Human siRNA Libraries (FlexiTube siRNA; commercially available from Qiagen) for host genes and used. In addition, AllStars Negative Control siRNA (commercially available from Qiagen) (a control siRNA) was used as a negative control. In addition, siRNA (GGA UCU UAU UUC UUC GGA GUU) of NP genes of WSN virus was purchased from Sigma Aldrich.

Specifically, first, an RNAiMAX Reagent (commercially available from Invitrogen) was used to transfect 2 types of siRNA into HEK293 cells at 25 nM (final concentration: 50 nM) twice.

### <Cell viability >

Viability of cells 24 hours after the second transfection of siRNA was determined according to the appended instructions of CellTiter-Glo assay system (commercially available from Promega Corporation). The ratio of the number of living cells among cells into which each siRNA was introduced to the number of living cells among cells into which the control siRNA was introduced was calculated as cell viability (%).

### <qRT-PCR>

Quantitative reverse transcription-PCR (qRT-PCR) was performed on cells before transfection of siRNA and cells 48 hours after transfection and it was confirmed whether expression of target host genes was suppressed due to siRNA.

Specifically, first, in the same manner as in the above <siRNA>, siRNA was transfected into HEK293 cells and cells 48 hours after the second transfection were lysed in the cell lysis buffer to prepare a lysate. Total RNA was extracted from the prepared lysate using the Maxwell 16 LEV simply RNA Tissue Kit (commercially available from Promega Corporation). A reverse transcription reaction was performed using ReverTra Ace qPCR RT Master Mix (commercially available from Toyobo Co., Ltd.) or SuperScript III Reverse Transcriptase (commercially available from Invitrogen) using the total RNA as a template. Using the synthesized cDNA as a template, a primer set specific to each host gene and THUNDERBIRD SYBR qPCR Mix (commercially available from Toyobo Co., Ltd.) were used to perform quantitative PCR. The relative mRNA expression levels of each host gene were calculated by the ΔΔCt method using β-actin as internal control. The ratio of an mRNA expression level in cells into which each siRNA was introduced to an mRNA expression level in cells into which the control siRNA was introduced was calculated as a knockdown efficiency (%).

### < Replicative competence of virus>

In the same manner as in <siRNA>, in two 24-well dishes, siRNA was transfected into HEK293 cells, and the cells after the second transfection were infected with an influenza virus of 50 pfu (plaque-forming units). A culture supernatant was collected 48 hours after the viral infection and virus titers were examined through a plaque assay using MDCK cells. A value obtained by dividing a common logarithmic value of a virus titer in cells into which each siRNA was introduced by a common logarithmic value of a virus titer in cells into which the control siRNA was introduced was calculated as an amount of change in virus titer.

As a result, in 323 host genes, gene expression levels were reduced due to transfection of siRNA. Among the 323 host genes, in 299 host genes, an influenza virus titer was reduced by a common logarithmic value of 2 or more (that is, an amount of change in virus titer-2 or more), and in 24 host genes, an influenza virus titer was increased by a common logarithmic value of 1 or more (that is, an amount of change in virus titer was 1 or more). In the following 91 host genes among the host genes, although cell viability of host cells remained at 80% or more, an influenza virus titer was reduced by a common logarithmic value of 3 or more, and thus they were indicated to be useful as a target of the anti-influenza virus agent: ANP32B gene, AP2A2 gene, ASCC3L1 gene, ATP50 gene, BAG3 gene, BASP1 gene, BRD8 gene, BUB3 gene, C14orf173 gene, C19orf43 gene, CAPRIN1 gene, CCDC135 gene, CCDC56 gene, CHERP gene, CIRBP gene, CLTC gene, CNOT1 gene, CTNNB1 gene, CYC1 gene, DDX21 gene, DDX55 gene, DKFZp564K142 gene, DNAJC11 gene, DPM3 gene, EEF1A2 gene, EEF2 gene, FAM73B gene, FLJ20303 gene, GBF1 gene, GEMIN4 gene, HNRNPK gene, IARS gene, IGF2BP2 gene, ITGA3 gene, ITGB4BP gene, ITM2B gene, JAK1 gene, KIAA0664 gene, KRT14 gene, LRPPRC gene, MRCL3 gene, MYH10 gene, NCAPD3 gene, NCLN gene, NDUFA10 gene, NDUFS8 gene, NFIA gene, NIBP gene, NUP160 gene, NUP205 gene, PCDHB12 gene, PHB gene, PPP6C gene, PSMA4 gene, PSMA5 gene, PSMB2 gene, PSMC1 gene, PSMC4 gene, PSMC6 gene, PSMD11 gene, PSMD12 gene, PSMD13 gene, PSMD14 gene, PSMD2 gene, PSMD6 gene, RCN1 gene, RPL26 gene, S100A4 gene, SAMHD1 gene, SDF2L1 gene, SDF4 gene, SF3A2 gene, SF3B2 gene, SF3B4 gene, SFRS10 gene, SFRS2B gene, SNRP70 gene, SNRPC gene, SNRPD3 gene, SQSTM1 gene, STK38 gene, TAF15 gene, TESC gene, THOC2 gene, TOMM40 gene, TRIM28 gene, UAP1 gene, USP9X gene, VCP gene, XPO1 gene, and ZC3H15 gene.

The amount of change in virus titer, the cell viability (%), and the knockdown efficiency (%) of the 91 host genes are shown in Tables 1 to 3.

**[Table 1]**

| Gene name | Gene ID | Amount of change in virus titer | Cell viability (%) | Knockdown efficiency (%) |
|---|---|---|---|---|
| ANP32B | 10541 | -4.65 | 99.45 | 1.56 |
| AP2A2 | 161 | -3.18 | 95.81 | 6.28 |
| ASCC3L1 | 23020 | -3.08 | 96.11 | 2.91 |
| ATP50 | 539 | -3.74 | 82.96 | 13.09 |
| BAG3 | 9531 | -4.11 | 103.25 | 5.07 |
| BASP1 | 10409 | -3.51 | 107.98 | 40.30 |
| BRD8 | 10902 | -4.86 | 114.74 | 48.14 |
| BUB3 | 9184 | -3.28 | 97.88 | 9.68 |
| C14orf173 | 64423 | -3.01 | 87.95 | 14.83 |
| C19orf43 | 79002 | -4.18 | 108.05 | 8.39 |
| CAPRIN1 | 4076 | -4.83 | 97.06 | 5.98 |
| CCDC135 | 84229 | -4.13 | 114.76 | 2.24 |
| CCDC56 | 28958 | -3.45 | 101.34 | 33.50 |
| CHERP | 10523 | -4.63 | 103.63 | 17.74 |
| CIRBP | 1153 | -3.07 | 114.17 | 10.46 |
| CLTC | 1213 | -3.11 | 95.80 | 4.45 |
| CNOT1 | 23019 | -3.02 | 119.11 | 6.63 |
| CTNNB1 | 1499 | -3.55 | 115.53 | 1.73 |
| CYC1 | 1537 | -3.66 | 94.25 | 3.10 |
| DDX21 | 9188 | -3.52 | 99.20 | 11.33 |
| DDX55 | 57696 | -3.37 | 97.39 | 24.07 |
| DKFZp564K142 | 84061 | -3.11 | 95.17 | 1.20 |
| DNAJC11 | 55735 | -3.14 | 96.26 | 35.77 |
| DPM3 | 54344 | -4.16 | 85.79 | 1.41 |
| EEF1A2 | 1917 | -3.15 | 91.40 | 1.67 |
| EEF2 | 1938 | -3.41 | 110.44 | 3.80 |
| FAM73B | 84895 | -3.79 | 111.63 | 0.12 |
| FLJ20303 | 54888 | -3.68 | 103.10 | 46.80 |
| GBF1 | 8729 | -5.06 | 109.48 | 9.48 |
| GEMIN4 | 50628 | -3.00 | 85.59 | 41.30 |

**[Table 2]**

| Gene name | Gene ID | Amount of change in virus titer | Cell viability (%) | Knockdown efficiency (%) |
|---|---|---|---|---|
| HNRNPK | 3190 | -4.99 | 113.48 | 1.12 |
| IARS | 3376 | -3.17 | 103.38 | 8.00 |
| IGF2BP2 | 10644 | -3.30 | 89.82 | 6.61 |
| ITGA3 | 3675 | -3.38 | 92.90 | 8.68 |
| ITGB4BP | 3692 | -3.27 | 81.34 | 26.05 |
| ITM2B | 9445 | -3.23 | 100.15 | 0.68 |
| JAK1 | 3716 | -5.10 | 80.07 | 1.94 |
| KIAA0664 | 23277 | -3.82 | 91.33 | 12.35 |
| KRT14 | 3861 | -3.66 | 108.94 | 17.36 |
| LRPPRC | 10128 | -4.16 | 108.87 | 8.33 |
| MRCL3 | 10627 | -3.11 | 92.84 | 2.00 |
| MYH10 | 4628 | -3.57 | 89.49 | 7.05 |
| NCAPD3 | 23310 | -3.14 | 87.57 | 30.04 |
| NCLN | 56926 | -3.38 | 90.16 | 3.79 |
| NDUFA10 | 4705 | -3.52 | 103.54 | 9.75 |
| NDUFS8 | 4728 | -3.49 | 92.94 | 1.38 |
| NFIA | 4774 | -4.34 | 98.05 | 3.00 |
| NIBP | 83696 | -3.59 | 98.42 | 8.65 |
| NUP160 | 23279 | -3.77 | 98.13 | 47.25 |
| NUP205 | 23165 | -4.71 | 91.59 | 27.45 |
| PCDHB12 | 56124 | -3.66 | 110.37 | 35.50 |
| PHB | 5245 | -5.89 | 86.57 | 1.56 |
| PPP6C | 5537 | -4.84 | 104.25 | 5.65 |
| PSMA4 | 5685 | -3.03 | 101.54 | 11.10 |
| PSMA5 | 5686 | -4.01 | 103.75 | 4.66 |
| PSMB2 | 5690 | -3.14 | 86.44 | 1.92 |
| PSMC1 | 5700 | -4.02 | 80.49 | 15.81 |
| PSMC4 | 5704 | -4.52 | 90.10 | 27.87 |
| PSMC6 | 5706 | -4.77 | 94.29 | 17.43 |
| PSMD11 | 5717 | -5.16 | 86.58 | 10.03 |

**[Table 3]**

| Gene name | Gene ID | Amount of change in virus titer | Cell viability (%) | Knockdown efficiency (%) |
|---|---|---|---|---|
| PSMD12 | 5718 | -3.41 | 109.54 | 8.30 |
| PSMD13 | 5719 | -3.28 | 102.30 | 6.80 |
| PSMD 14 | 10213 | -4.09 | 83.53 | 15.39 |
| PSMD2 | 5708 | -3.66 | 83.48 | 14.71 |
| PSMD6 | 9861 | -3.91 | 81.82 | 17.91 |
| RCN1 | 5954 | -3.08 | 83.94 | 3.42 |
| RPL26 | 6154 | -3.14 | 89.03 | 9.93 |
| S100A4 | 6275 | -3.66 | 106.05 | 45.83 |
| SAMHD1 | 25939 | -3.48 | 101.14 | 34.03 |
| SDF2L1 | 23753 | -4.45 | 91.54 | 0.82 |
| SDF4 | 51150 | -3.44 | 92.11 | 6.35 |
| SF3A2 | 8175 | -3.13 | 89.26 | 16.05 |
| SF3B2 | 10992 | -3.06 | 82.55 | 17.71 |
| SF3B4 | 10262 | -4.33 | 102.63 | 5.89 |
| SFRS10 | 6434 | -4.92 | 105.62 | 32.28 |
| SFRS2B | 10929 | -3.25 | 94.42 | 29.98 |
| SNRP70 | 6625 | -3.30 | 83.21 | 7.73 |
| SNRPC | 6631 | -3.23 | 110.27 | 2.95 |
| SNRPD3 | 6634 | -4.02 | 82.34 | 0.83 |
| SQSTM1 | 8878 | -3.41 | 99.11 | 18.46 |
| STK38 | 11329 | -4.63 | 93.74 | 1.93 |
| TAF15 | 8148 | -3.65 | 106.52 | 0.72 |
| TESC | 54997 | -4.34 | 104.88 | 8.30 |
| THOC2 | 57187 | -4.39 | 123.42 | 7.04 |
| TOMM40 | 10452 | -3.33 | 108.53 | 2.04 |
| TRIM28 | 10155 | -3.58 | 98.94 | 12.60 |
| UAP1 | 6675 | -3.01 | 106.91 | 44.47 |
| USP9X | 8239 | -3.37 | 112.88 | 14.23 |
| VCP | 7415 | -3.11 | 86.85 | 5.14 |
| XPO1 | 7514 | -4.91 | 102.74 | 17.20 |
| ZC3H15 | 55854 | -5.28 | 98.39 | 4.03 |

### <Influence on intracellular protein synthesis>

It was examined whether suppression of expression of these 91 host genes influenced intracellular protein synthesis.

Specifically, in the same manner as in <siRNA>, siRNA was transfected into HEK293 cells and a plasmid for expressing Renilla luciferase under control of an RNA Polymerase II promotor (for example, chicken β-actin promotor) that functioned within a cell was used as a control in cells 24 hours after the second transfection.

A luciferase assay was performed on cells 48 hours after the transfection using a Renilla Luciferase Assay System (commercially available from Promega Corporation). Luciferase activity was measured using the GloMax-96 Microplate Luminometer (commercially available from Promega Corporation).

The ratio of Renilla luciferase activity of cells into which each siRNA was introduced to Renilla luciferase activity of cells into which the control siRNA was introduced was calculated as a synthesis efficiency (%) of the intracellular protein. The calculated synthesis efficiency (%) of the intracellular proteins is shown in Table 4. As a result, firefly luciferase activity in cells into which siRNA of 28 host genes (ATP50 gene, CNOT1 gene, GEMIN4 gene, ITGB4BP gene, NCAPD3 gene, NUP160 gene, NUP205 gene, PSMA4 gene, PSMA5 gene, PSMB2 gene, PSMC1 gene, PSMC4 gene, PSMD11 gene, PSMD2 gene, PSMD6 gene, SF3A2 gene, SF3B4 gene, SNRPD3 gene, VCP gene, PSMC6 gene, PSMD12 gene, PSMD14 gene, SAMHD1 gene, SF3B2 gene, SNRP70 gene, CAPRIN1 gene, PHB gene, and SFRS10 gene) among the 91 host genes was introduced was decreased 80% or more (p<0.05) compared to that of cells into which the control siRNA was introduced. The results indicate that these host genes were involved in transcription and translation in host cells, and transcription and translation of an influenza virus were suppressed and influenza virus replication was inhibited by decreasing expression of these host genes,.

**[Table 4]**

| Synthesis efficiency (%) of host proteins | | | | | |
|---|---|---|---|---|---|
| Gene name | Activity (%) | Gene name | Activity (%) | Gene name | Activity (%) |
| AP2A2 | 75.11 | KIAA0664 | 60.55 | TRIM28 | 37.64 |
| ASCC3L1 | 65.37 | KRT14 | 26.22 | UAP1 | 80.46 |
| ATP50 | 2.79 | MRCL3 | 134.23 | USP9X | 202.02 |
| BAG3 | 72.00 | MYH10 | 96.54 | VCP | 2.27 |
| BRD8 | 146.60 | NCAPD3 | 4.02 | ZC3H15 | 71.50 |
| BUB3 | 50.58 | NCLN | 135.47 | BASP1 | 84.14 |
| C19orf43 | 101.04 | NDUFS8 | 88.55 | C14orf173 | 77.71 |
| CCDC135 | 72.77 | NIBP | 135.35 | CTNNB1 | 164.22 |
| CCDC56 | 112.47 | NUP160 | 9.04 | PSMC6 | 9.36 |
| CHERP | 172.80 | NUP205 | 3.97 | PSMD12 | 3.67 |
| CIRBP | 69.48 | PSMA4 | 10.32 | PSMD14 | 0.95 |
| CLTC | 22.74 | PSMA5 | 4.23 | SAMHD1 | 10.13 |
| CNOT1 | 10.93 | PSMB2 | 1.47 | SF3B2 | 1.99 |
| CYC1 | 48.89 | PSMC1 | 6.49 | SNRP70 | 4.52 |
| DDX21 | 92.65 | PSMC4 | 3.33 | THOC2 | 22.91 |
| DDX55 | 38.81 | PSMD11 | 1.82 | XPO1 | 20.42 |
| DKFZp564K142 | 116.60 | PSMD13 | 23.75 | ANP32B | 45.32 |
| DNAJC11 | 89.91 | PSMD2 | 1.55 | CAPRIN1 | 5.42 |
| DPM3 | 22.56 | PSMD6 | 0.30 | LRPPRC | 24.48 |
| EEF1A2 | 89.30 | RPL26 | 32.18 | NFIA | 54.13 |
| EEF2 | 67.89 | S100A4 | 109.39 | PHB | 8.83 |
| FAM73B | 47.30 | SDF2L1 | 40.47 | PPP6C | 39.93 |
| FLJ20303 | 39.03 | SDF4 | 116.39 | SFRS10 | 10.82 |
| GBF1 | 67.13 | SF3A2 | 2.81 | STK38 | 34.02 |
| GEMIN4 | 12.89 | SF3B4 | 2.77 | TESC | 30.75 |
| HNRNPK | 105.55 | SFRS2B | 112.74 | JAK1 | 83.80 |
| IARS | 114.19 | SNRPC | 87.85 | PCDHB12 | 77.64 |
| IGF2BP2 | 151.66 | SNRPD3 | 5.34 | NDUFA10 | 102.53 |
| ITGA3 | 212.86 | SQSTM1 | 34.14 | RCN1 | 40.47 |
| ITGB4BP | 9.07 | TAF15 | 192.11 | | |
| ITM2B | 137.49 | TOMM40 | 125.32 | | |

### <Mini-replicon assay>

Determination of whether the 91 host genes were involved in virus genome replication and transcription was examined using a mini-replicon assay (refer to Non Patent Literature 10) through which the activity of the influenza viral RNA Polymerase was examined. More specifically, the mini-replicon assay is an assay in which the activity of the viral replication complex (the complex containing the PB2 protein, the PB1 protein, the PA protein, and the NP protein) is examined based on replicative activity of virus-like RNA encoding a firefly luciferase reporter protein.

Specifically, in the same manner as in <siRNA>, siRNA was transfected into HEK293 cells and a plasmid for expressing the PA, a plasmid for expressing the PB1, a plasmid for expressing the PB2, a plasmid for expressing the NP, and a plasmid (pPolINP(0)luc2(0)) for expressing the virus-like RNA that encoding firefly luciferase were transfected into cells 24 hours after the second transfection. Also, the plasmid for expressing the PA, PB1, PB2 or NP was obtained by integrating cDNA that encodes each protein in a multi cloning site of plasmid pCAGGS. In addition, a plasmid for expressing Renilla luciferase under control of an RNA Polymerase II promotor (for example, a chicken β-actin promotor) that functions within a host cell was used as a control.

A luciferase assay was performed on cells 48 hours after the transfection using the Dual-Glo Luciferase assay system (commercially available from Promega Corporation). Luciferase activity was measured using the GloMax-96 Microplate Luminometer (commercially available from Promega Corporation). Virus RNA Polymerase activity was calculated as firefly luciferase activity that was normalized by Renilla luciferase activity.

The ratio of firefly luciferase activity of cells into which each siRNA was introduced to firefly luciferase activity of cells into which the control siRNA was introduced was calculated as viral polymerase activity (%). This viral polymerase activity indicates an expression level of the firefly luciferase reporter protein and is an indicator of efficiency of virus genome replication and transcription. The calculated viral polymerase activity (%) is shown in Table 5. As a result, viral polymerase activity in cells into which siRNA of 9 host genes (BUB3 gene, CCDC56 gene, CLTC gene, CYC1 gene, NIBP gene, ZC3H15 gene, C14orf173 gene, CTNNB1 gene, and ANP32B gene) among the 91 host genes was introduced, that is, virus genome replication and transcription, was decreased 50% or more (p<0.05) compared to that of cells into which the control siRNA was introduced. The results indicate that these host genes were involved in virus genome replication and transcription and genome replication and transcription of an influenza virus were suppressed by decreasing expression of these host genes.

**[Table 5]**

| Viral polymerase activity (%) | | | | | |
|---|---|---|---|---|---|
| Gene name | Activity (%) | Gene name | Activity (%) | Gene name | Activity (%) |
| AP2A2 | 137.72 | KIAA0664 | 125.03 | TRIM28 | 113.41 |
| ASCC3L1 | 55.38 | KRT14 | 126.87 | UAP1 | 83.45 |
| ATP50 | 87.21 | MRCL3 | 82.07 | USP9X | 179.66 |
| BAG3 | 212.58 | MYH10 | 133.56 | VCP | 132.26 |
| BRD8 | 134.18 | NCAPD3 | 75.26 | ZC3H15 | 16.67 |
| BUB3 | 29.86 | NCLN | 67.85 | BASP1 | 65.72 |
| C19orf43 | 60.22 | NDUFS8 | 62.68 | C14orf173 | 31.32 |
| CCDC135 | 164.75 | NIBP | 32.54 | CTNNB1 | 41.41 |
| CCDC56 | 35.89 | NUP160 | 161.95 | PSMC6 | 68.22 |
| CHERP | 50.76 | NUP205 | 185.48 | PSMD12 | 83.21 |
| CIRBP | 125.42 | PSMA4 | 78.06 | PSMD14 | 135.56 |
| CLTC | 21.45 | PSMA5 | 152.59 | SAMHD 1 | 112.95 |
| CNOT1 | 122.58 | PSMB2 | 186.22 | SF3B2 | 89.83 |
| CYC1 | 39.77 | PSMC1 | 60.30 | SNRP70 | 116.15 |
| DDX21 | 227.00 | PSMC4 | 123.01 | THOC2 | 432.48 |
| DDX55 | 243.36 | PSMD11 | 105.10 | XPO1 | 345.49 |
| DKFZp564K142 | 73.46 | PSMD13 | 87.38 | ANP32B | 20.97 |
| DNAJC11 | 115.60 | PSMD2 | 168.78 | CAPRIN 1 | 240.52 |
| DPM3 | 166.79 | PSMD6 | 165.82 | LRPPRC | 99.80 |
| EEF1A2 | 120.27 | RPL26 | 75.28 | NFIA | 103.74 |
| EEF2 | 56.88 | S100A4 | 108.15 | PHB | 482.29 |
| FAM73B | 148.67 | SDF2L1 | 76.28 | PPP6C | 337.17 |
| FLJ20303 | 73.70 | SDF4 | 60.57 | SFRS10 | 143.36 |
| GBF1 | 218.22 | SF3A2 | 395.26 | STK38 | 181.90 |
| GEMIN4 | 80.46 | SF3B4 | 139.22 | TESC | 113.84 |
| HNRNPK | 220.87 | SFRS2B | 67.87 | JAK1 | 170.25 |
| IARS | 119.39 | SNRPC | 134.40 | PCDHB12 | 230.68 |
| IGF2BP2 | 96.58 | SNRPD3 | 173.08 | NDUFA10 | 135.21 |
| ITGA3 | 57.93 | SQSTM1 | 136.86 | RCN1 | 228.41 |
| ITGB4BP | 142.67 | TAF15 | 60.96 | | |
| ITM2B | 136.82 | TOMM40 | 221.22 | | |

### <PB2-KO/Rluc virus assay>

In order to examine whether the 91 host genes were involved at an early stage of the virus life cycle, a PB2-KO/Rluc virus assay was performed and virus invasion efficiency was examined.

Specifically, in the same manner as in <siRNA>, siRNA was transfected into HEK293 cells and PB2-KO/Rluc virus was infected into cells 24 hours after the second transfection. A luciferase assay was performed on cells 8 hours after the infection using a Renilla luciferase assay system (commercially available from Promega Corporation). Fluorescence was measured using the GloMax-96 Microplate Luminometer (commercially available from Promega Corporation).

A ratio of Renilla luciferase activity of cells into which each siRNA was introduced to Renilla luciferase activity of cells into which the control siRNA was introduced was calculated as virus invasion efficiency (%). The calculated virus invasion efficiency (%) is shown in Table 6. As a result, virus invasion efficiency of 23 host genes (SF3A2 gene, GEMIN4 gene, SFRS10 gene, BAG3 gene, CAPRIN1 gene, CCDC135 gene, IGF2BP2 gene, KRT14 gene, ATP50 gene, SAMHD1 gene, PSMD6 gene, BRD8 gene, PSMD11 gene, SF3B2 gene, SF3B4 gene, DPM3 gene, NCAPD3 gene, EEF2 gene, PHB gene, NUP205 gene, S100A4 gene, PSMD14 gene, and DDX55 gene) among the 91 host genes was decreased 50% or more (p<0.05) compared to that of cells into which the control siRNA was introduced. The results indicate that these host genes were involved in invasion of an influenza virus into host cells, and invasion of an influenza virus into host cells and influenza virus replication were inhibited by decreasing expression of these host genes.

**[Table 6]**

| Virus invasion efficiency (%) | | | | | |
|---|---|---|---|---|---|
| Gene name | Activity (%) | Gene name | Activity (%) | Gene name | Activity (%) |
| AP2A2 | 105.13 | KIAA0664 | 368.93 | TRIM28 | 57.02 |
| ASCC3L1 | 127.45 | KRT14 | 29.12 | UAP1 | 101.45 |
| ATP5O | 32.22 | MRCL3 | 66.72 | USP9X | 69.50 |
| BAG3 | 26.26 | MYH10 | 127.85 | VCP | 139.02 |
| BRD8 | 36.25 | NCAPD3 | 37.22 | ZC3H15 | 61.72 |
| BUB3 | 111.12 | NCLN | 102.04 | BASP1 | 81.97 |
| C19orf43 | 72.52 | NDUFS8 | 89.39 | C14orf173 | 52.52 |
| CCDC135 | 26.41 | NIBP | 157.58 | CTNNB1 | 107.63 |
| CCDC56 | 135.37 | NUP160 | 78.57 | PSMC6 | 72.44 |
| CHERP | 152.40 | NUP205 | 38.39 | PSMD12 | 70.67 |
| CIRBP | 81.17 | PSMA4 | 141.94 | PSMD14 | 41.13 |
| CLTC | 130.96 | PSMA5 | 61.95 | SAMHD1 | 34.45 |
| CNOT1 | 99.07 | PSMB2 | 58.19 | SF3B2 | 36.43 |
| CYC1 | 208.99 | PSMC1 | 170.15 | SNRP70 | 79.70 |
| DDX21 | 152.99 | PSMC4 | 111.62 | THOC2 | 74.46 |
| DDX55 | 41.36 | PSMD11 | 36.26 | XPO1 | 56.05 |
| DKFZp564K142 | 61.95 | PSMD13 | 128.58 | ANP32B | 77.65 |
| DNAJC11 | 89.42 | PSMD2 | 122.48 | CAPRIN 1 | 26.29 |
| DPM3 | 37.04 | PSMD6 | 35.49 | LRPPRC | 117.05 |
| EEF1A2 | 118.58 | RPL26 | 53.03 | NFIA | 143.19 |
| EEF2 | 37.31 | S100A4 | 40.97 | PHB | 37.52 |
| FAM73B | 72.07 | SDF2L1 | 87.44 | PPP6C | 50.69 |
| FLJ20303 | 171.60 | SDF4 | 111.77 | SFRS10 | 26.23 |
| GBF1 | 110.84 | SF3A2 | 16.50 | STK38 | 90.17 |
| GEMIN4 | 19.22 | SF3B4 | 36.86 | TESC | 91.50 |
| HNRNPK | 89.22 | SFRS2B | 62.16 | JAK1 | 234.62 |
| IARS | 122.66 | SNRPC | 192.30 | PCDHB12 | 80.80 |
| IGF2BP2 | 27.46 | SNRPD3 | 189.24 | NDUFA10 | 58.58 |
| ITGA3 | 148.66 | SQSTM1 | 70.37 | RCN1 | 89.00 |
| ITGB4BP | 58.65 | TAF15 | 182.19 | | |
| ITM2B | 84.31 | TOMM40 | 74.91 | | |

In addition, it was found that, among these 23 host genes, 9 host genes (BAG3 gene, BRD8 gene, CCDC135 gene, DDX55 gene, DPM3 gene, EEF2 gene, IGF2BP2 gene, KRT14 gene, and S100A4 gene) were not involved in transcription and translation of host cells and were specifically involved in transcription and translation of an influenza virus. In addition, in these 9 host genes, influenza virus replication inhibitory activity was not confirmed in the mini-replicon assay. Therefore, the results indicate that these host genes played important roles at the early stage of the virus life cycle such as binding of viruses into surfaces of host cells, incorporation into host cells, and transition of a viral ribonucleoprotein (vRNP) complex into the nucleus.

### <VLP formation assay>

Determination of whether the 91 host genes were involved in virus particle formation was examined through a virus-like particle (VLP) formation assay.

Specifically, in the same manner as in <siRNA>, in HEK293 cells transfected with siRNA, a plasmid for expressing the HA, a plasmid for expressing the NA, and a plasmid for expressing the M1 were transfected using a TransIT293 transfection reagent (commercially available from Mirus). Also, the plasmid for expressing the HA, NA, or M1 was obtained by integrating cDNA that encodes each protein in a multi cloning site of plasmid pCAGGS.

Cells 48 hours after the plasmid transfection were lysed in an SDS sample buffer solution (commercially available from Wako Pure Chemical Industries, Ltd.) containing 100 mM DTT. The obtained lysate was collected and subjected to a centrifugation treatment (3000×g, 4°C, 5 minutes), and a supernatant containing VLP was separated from cell residues and collected. The obtained supernatant was placed on phosphate buffered saline (PBS) containing 30 mass/volume% sucrose put into an ultracentrifugation tube and subjected to an ultracentrifugation treatment (50000 rpm, 4°C, 1 hour, SW55Ti Rotor). The obtained precipitate was lysed in an SDS sample buffer solution (commercially available from Wako Pure Chemical Industries, Ltd.) containing 100 mM DTT to prepare a western blotting sample.

A sample in which a Tris-Glycine SDS sample buffer (commercially available from Invitrogen) was mixed in the prepared western blotting sample was applied to 4%-20% Mini-PROTEAN TGX gradient gels (commercially available from Bio-Rad Laboratories, Inc) and was subjected to SDS-PAGE. The separated proteins were transcribed into a PVDF membrane, and the transcription membrane was blocked using a Blocking One solution (commercially available from Nakarai Tesque). The transcription membrane was incubated in a primary antibody solution (a solution of rabbit anti-WSN virus antibody (R309) or anti β-actin (AC-74) antibody was diluted in a solution (solution I) included in a Can Get Signal (commercially available from Toyobo Co., Ltd.)) at room temperature for at least 1 hour. Next, the transcription membrane was washed with TBS (TBST) containing 0.05 volume% Tween-20 three times. Then, a secondary antibody solution (a solution of ECL donkey anti-mouse IgG antibodies (commercially available from GE healthcare) conjugated with horseradish peroxidase was diluted in a solution (solution II) included in a Can Get Signal (commercially available from Toyobo Co., Ltd.)) was incubated and then washed with TBST three times. The transcription membrane was incubated in an ECL Prime Western blotting detection reagent (commercially available from GE healthcare) and a chemiluminescent signal was then detected in bands of the HA protein and the M1 protein in VLPs and bands of β-actin using a VersaDoc Imaging System (commercially available from Bio-Rad Laboratories, Inc).

An amount of VLPs produced was calculated as the ratio of an amount of the HA protein or the M1 protein in VLPs with respect to an amount of the HA protein or the M1 protein in the lysate. The ratio of an amount of VLPs produced in cells into which each siRNA was introduced to an amount of VLPs produced in cells into which the control siRNA was introduced was calculated as a production efficiency (%) of VLPs. The result of the VLP production efficiency (%) based on the HA protein is shown in Table 7, and the result of the VLP production efficiency (%) based on the M1 protein is shown in Table 8. As a result, a VLP production efficiency of 15 host genes (ASCC3L1 gene, BRD8 gene, C19orf43 gene, DDX55 gene, DKFZp564K142 gene, DPM3 gene, EEF2 gene, FAM73B gene, FLJ20303 gene, GBF1 gene, NCLN gene, C14orf173 gene, XPO1 gene, LRPPRC gene, and RCN1 gene) among the 91 host genes was decreased 50% or more (p<0.05) compared to that of cells into which the control siRNA was introduced. The results indicate that these host genes were involved in formation of VLP, and formation of VLP and influenza virus replication were inhibited by decreasing expression of these host genes.

**[Table 7]**

| VLP production efficiency (%) based on HA proteins | | | | | |
|---|---|---|---|---|---|
| Gene name | Activity (%) | Gene name | Activity (%) | Gene name | Activity (%) |
| AP2A2 | 177.34 | KIAA0664 | 116.85 | TRIM28 | 111.28 |
| ASCC3L1 | 164.31 | KRT14 | 121.78 | UAP1 | 96.46 |
| ATP50 | 231.41 | MRCL3 | 257.17 | USP9X | 113.07 |
| BAG3 | 76.58 | MYH10 | 144.03 | VCP | 251.95 |
| BRD8 | 131.03 | NCAPD3 | 60.43 | ZC3H15 | 346.37 |
| BUB3 | 141.86 | NCLN | 49.60 | BASP1 | 198.63 |
| C19orf43 | 138.87 | NDUFS8 | 82.87 | C14orf173 | 63.05 |
| CCDC135 | 80.11 | NIBP | 88.27 | CTNNB1 | 115.18 |
| CCDC56 | 99.94 | NUP160 | 91.43 | PSMC6 | 138.70 |
| CHERP | 139.28 | NUP205 | 87.21 | PSMD 12 | 173.40 |
| CIRBP | 206.25 | PSMA4 | 109.43 | PSMD14 | 200.50 |
| CLTC | 183.70 | PSMA5 | 81.34 | SAMHD1 | 134.99 |
| CNOT1 | 113.66 | PSMB2 | 209.71 | SF3B2 | 200.77 |
| CYC1 | 145.24 | PSMC1 | 135.75 | SNRP70 | 404.20 |
| DDX21 | 89.03 | PSMC4 | 212.29 | THOC2 | 174.33 |
| DDX55 | 62.07 | PSMD11 | 149.91 | XPO1 | 61.99 |
| DKFZp564K142 | 77.20 | PSMD13 | 94.54 | ANP32B | 206.57 |
| DNAJC11 | 98.32 | PSMD2 | 134.75 | CAPRIN 1 | 75.64 |
| DPM3 | 37.43 | PSMD6 | 125.70 | LRPPRC | 72.43 |
| EEF1A2 | 73.32 | RPL26 | 88.86 | NFIA | 97.73 |
| EEF2 | 32.28 | S100A4 | 143.16 | PHB | 69.14 |
| FAM73B | 22.48 | SDF2L1 | 219.96 | PPP6C | 79.39 |
| FLJ20303 | 24.25 | SDF4 | 363.99 | SFRS10 | 82.25 |
| GBF1 | 46.44 | SF3A2 | 411.61 | STK38 | 295.34 |
| GEMIN4 | 38.02 | SF3B4 | 693.20 | TESC | 85.68 |
| HNRNPK | 60.20 | SFRS2B | 229.98 | JAK1 | 74.23 |
| IARS | 117.45 | SNRPC | 161.92 | PCDHB12 | 82.21 |
| IGF2BP2 | 145.77 | SNRPD3 | 264.49 | NDUFA10 | 115.30 |
| ITGA3 | 108.10 | SQSTM1 | 123.78 | RCN1 | 117.61 |
| ITGB4BP | 105.61 | TAF15 | 119.20 | | |
| ITM2B | 152.06 | TOMM40 | 119.26 | | |

**[Table 8]**

| VLP production efficiency (%) based on M1 proteins | | | | | |
|---|---|---|---|---|---|
| Gene name | Activity (%) | Gene name | Activity (%) | Gene name | Activity (%) |
| AP2A2 | 105.25 | KIAA0664 | 65.31 | TRIM28 | 124.46 |
| ASCC3L1 | 37.96 | KRT14 | 65.35 | UAP1 | 129.75 |
| ATP50 | 30.59 | MRCL3 | 362.33 | USP9X | 233.73 |
| BAG3 | 54.31 | MYH10 | 260.58 | VCP | 239.19 |
| BRD8 | 46.15 | NCAPD3 | 246.13 | ZC3H15 | 332.94 |
| BUB3 | 77.36 | NCLN | 25.09 | BASP1 | 88.45 |
| C19orf43 | 31.44 | NDUFS8 | 126.10 | C14orf173 | 49.67 |
| CCDC135 | 65.85 | NIBP | 211.85 | CTNNB1 | 93.10 |
| CCDC56 | 84.01 | NUP160 | 75.36 | PSMC6 | 76.21 |
| CHERP | 86.77 | NUP205 | 49.40 | PSMD12 | 159.72 |
| CIRBP | 192.75 | PSMA4 | 56.67 | PSMD14 | 139.36 |
| CLTC | 452.07 | PSMA5 | 93.31 | SAMHD 1 | 86.38 |
| CNOT1 | 180.01 | PSMB2 | 67.65 | SF3B2 | 113.47 |
| CYC1 | 353.99 | PSMC1 | 39.83 | SNRP70 | 232.69 |
| DDX21 | 68.64 | PSMC4 | 84.63 | THOC2 | 52.38 |
| DDX55 | 34.18 | PSMD11 | 42.69 | XPO1 | 22.30 |
| DKFZp564K142 | 24.61 | PSMD13 | 51.04 | ANP32B | 157.69 |
| DNAJC11 | 66.02 | PSMD2 | 88.15 | CAPRIN 1 | 33.65 |
| DPM3 | 28.47 | PSMD6 | 63.50 | LRPPRC | 41.86 |
| EEF1A2 | 54.03 | RPL26 | 93.69 | NFIA | 98.23 |
| EEF2 | 75.49 | S100A4 | 96.92 | PHB | 29.04 |
| FAM73B | 95.69 | SDF2L1 | 78.78 | PPP6C | 87.35 |
| FLJ20303 | 34.39 | SDF4 | 114.31 | SFRS10 | 34.02 |
| GBF1 | 52.00 | SF3A2 | 91.21 | STK38 | 188.37 |
| GEMIN4 | 34.51 | SF3B4 | 87.39 | TESC | 59.33 |
| HNRNPK | 72.32 | SFRS2B | 75.66 | JAK1 | 57.70 |
| IARS | 196.77 | SNRPC | 70.65 | PCDHB 12 | 105.78 |
| IGF2BP2 | 101.08 | SNRPD3 | 56.78 | NDUFA10 | 89.91 |
| ITGA3 | 108.01 | SQSTM1 | 91.41 | RCN1 | 30.55 |
| ITGB4BP | 66.69 | TAF15 | 121.08 | | |
| ITM2B | 114.84 | TOMM40 | 130.27 | | |

### <Efficiency of incorporation of vRNP into progeny virus particles>

In order to examine whether the 91 host genes were involved in incorporation of vRNP into progeny virus particles, incorporation of vRNA and NP into progeny virus particles was examined.

Specifically, first, in the same manner as in <siRNA>, siRNA was transfected into HEK293 cells and cells after the second transfection were infected with an influenza virus with a multiplicity of infection (MOI) of 5. A culture supernatant containing released virus particles was separated from cell residues through a centrifugation treatment (3000xg, 4 °C, 5 minutes) and collected 12 hours after the infection. The obtained supernatant was placed on PBS containing 30 weight/volume% sucrose put into an ultracentrifugation tube and was subjected to an ultracentrifugation treatment (50000 rpm, 4 °C, 1 hour, SW55Ti Rotor). The precipitate containing virus particles was homogenized in PBS and viral RNA was extracted using the Maxwell 16 LEV simply RNA Tissue Kit. An amount of viral RNA in the supernatant and an amount of viral RNA in cells were quantified through strand specific real time PCR according to Kawakami's method (refer to Non Patent Literature 11). Also, a reverse transcription reaction using total RNA as a template was performed using SuperScript III Reverse Transcriptase and an influenza virus genome specific primer (vRNA tag_NP_1F; ggccgtcatggtggcgaatAGCAAAAGCAGGGTAGATAATCACTC (the lower case part is a tag sequence)) to which a tag sequence including 19 bases was added to the 5' terminus. In addition, quantitative PCR was performed using a primer specific to the tag sequence (vRNA tag; GGCCGTCATGGTGGCGAAT), a primer specific to a virus genome (WSN-NP_100R; GTTCTCCATCAGTCTCCATC), a probe labeled with 6-FAM/ZEN/IBFQ (IDT, WSN-NP_46-70; ATGGCGACCAAAGGCACCAAACGAT), and THUNDERBIRD Probe qPCR Mix.

An amount of vRNA and NP protein incorporated into progeny virus particles was determined by a value obtained by dividing an amount of vRNA or NP proteins in the viruses collected from the culture supernatant by an amount of vRNA or NP proteins in the lysate. The ratio of an amount of vRNA incorporated into progeny virus particles of cells into which each siRNA was introduced to an amount of vRNA incorporated into progeny virus particles of cells into which the control siRNA was introduced was calculated as vRNA incorporation efficiency (%). The ratio of an amount of the NP protein incorporated into progeny virus particles of cells into which each siRNA was introduced to an amount of the NP protein incorporated into progeny virus particles of cells into which the control siRNA was introduced was calculated as the NP protein incorporation efficiency (%). The calculation results are shown in Tables 9 and 10. As a result, among the 91 host genes, efficiency of vRNA incorporated into progeny virus particles in cells into which siRNA of 16 host genes (HNRNPK gene, DDX21 gene, JAK1 gene, EEF1A2 gene, SFRS2B gene, DNAJC11 gene, SQSTM1 gene, BASP1 gene, PCDHB 12 gene, KIAA0664 gene, SNRPC gene, PPP6C gene, MRCL3 gene, ITM2B gene, TAF15 gene, and SDF4 gene) was introduced was decreased 50% or more (p<0.05) compared to that of cells into which the control siRNA was introduced and efficiency of the NP protein incorporated into progeny virus particles in cells into which siRNA of 27 host genes (SFRS2B gene, BASP1 gene, THOC2 gene, SNRPC gene, KIAA0664 gene, PPP6C gene, HNRNPK gene, ITM2B gene, SQSTM1 gene, RPL26 gene, NDUFS8 gene, SDF2L1 gene, JAK1 gene, DDX21 gene, EEF1A2 gene, TRIM28 gene, SDF4 gene, USP9X gene, PSMD13 gene, TAF15 gene, CIRBP gene, CHERP gene, TESC gene, MYH10 gene, TOMM40 gene, MRCL3 gene, and PCDHB12 gene) was introduced was decreased 50% or more (p<0.05) compared to that of cells into which the control siRNA was introduced. The results indicate that these host genes were involved in incorporation of vRNA or NP proteins into progeny virus particles, and incorporation of vRNA or NP proteins into progeny virus particles was suppressed and influenza virus replication was inhibited by decreasing expression of these host genes.

**[Table 9]**

| Efficiency (%) of incorporation of vRNA into progeny virus | | | | | |
|---|---|---|---|---|---|
| Gene name | Activity (%) | Gene name | Activity (%) | Gene name | Activity (%) |
| AP2A2 | 64.90 | KIAA0664 | 37.10 | TRIM28 | 56.47 |
| ASCC3L1 | N/A | KRT14 | N/A | UAP1 | 73.58 |
| ATP50 | N/A | MRCL3 | 41.24 | USP9X | 51.30 |
| BAG3 | N/A | MYH10 | 102.84 | VCP | N/A |
| BRD8 | N/A | NCAPD3 | N/A | ZC3H15 | N/A |
| BUB3 | N/A | NCLN | N/A | BASP1 | 34.55 |
| C19orf43 | N/A | NDUFS8 | 59.61 | C14orf173 | N/A |
| CCDC135 | N/A | NIBP | N/A | CTNNB1 | N/A |
| CCDC56 | N/A | NUP160 | N/A | PSMC6 | N/A |
| CHERP | 60.18 | NUP205 | N/A | PSMD12 | N/A |
| CIRBP | 100.55 | PSMA4 | N/A | PSMD14 | N/A |
| CLTC | N/A | PSMA5 | N/A | SAMHD1 | N/A |
| CNOT1 | N/A | PSMB2 | N/A | SF3B2 | N/A |
| CYC1 | N/A | PSMC1 | N/A | SNRP70 | N/A |
| DDX21 | 22.01 | PSMC4 | N/A | THOC2 | 69.69 |
| DDX55 | N/A | PSMD11 | N/A | XPO1 | N/A |
| DKFZp564K142 | N/A | PSMD13 | 77.25 | ANP32B | N/A |
| DNAJC11 | 33.98 | PSMD2 | N/A | CAPRIN1 | N/A |
| DPM3 | N/A | PSMD6 | N/A | LRPPRC | N/A |
| EEF1A2 | 30.38 | RPL26 | 69.12 | NFIA | 114.66 |
| EEF2 | N/A | S100A4 | N/A | PHB | N/A |
| FAM73B | N/A | SDF2L1 | 50.50 | PPP6C | 40.69 |
| FLJ20303 | N/A | SDF4 | 48.77 | SFRS10 | N/A |
| GBF1 | N/A | SF3A2 | N/A | STK38 | 105.10 |
| GEMIN4 | N/A | SF3B4 | N/A | TESC | 65.09 |
| HNRNPK | 7.61 | SFRS2B | 33.34 | JAK1 | 29.55 |
| IARS | 55.35 | SNRPC | 38.36 | PCDHB12 | 35.01 |
| IGF2BP2 | N/A | SNRPD3 | N/A | NDUFA10 | 103.50 |
| ITGA3 | 104.80 | SQSTM1 | 34.21 | RCN1 | N/A |
| ITGB4BP | N/A | TAF15 | 44.19 | | |
| ITM2B | 43.04 | TOMM40 | 62.85 | | |

**[Table 10]**

| Efficiency (%) of incorporation of NP proteins into progeny virus | | | | | |
|---|---|---|---|---|---|
| Gene name | Activity (%) | Gene name | Activity (%) | Gene name | Activity (%) |
| AP2A2 | 100.03 | KIAA0664 | 17.38 | TRIM28 | 27.83 |
| ASCC3L1 | N/A | KRT14 | N/A | UAP1 | 53.41 |
| ATP50 | N/A | MRCL3 | 44.54 | USP9X | 29.00 |
| BAG3 | N/A | MYH10 | 42.45 | VCP | N/A |
| BRD8 | N/A | NCAPD3 | N/A | ZC3H15 | N/A |
| BUB3 | N/A | NCLN | N/A | BASP1 | 11.72 |
| C19orf43 | N/A | NDUFS8 | 21.10 | C14orf173 | N/A |
| CCDC135 | N/A | NIBP | N/A | CTNNB1 | N/A |
| CCDC56 | N/A | NUP160 | N/A | PSMC6 | N/A |
| CHERP | 35.95 | NUP205 | N/A | PSMD12 | N/A |
| CIRBP | 35.27 | PSMA4 | N/A | PSMD 14 | N/A |
| CLTC | N/A | PSMA5 | N/A | SAMHD1 | N/A |
| CNOT1 | N/A | PSMB2 | N/A | SF3B2 | N/A |
| CYC1 | N/A | PSMC1 | N/A | SNRP70 | N/A |
| DDX21 | 24.15 | PSMC4 | N/A | THOC2 | 15.61 |
| DDX55 | N/A | PSMD11 | N/A | XPO1 | N/A |
| DKFZp564K142 | N/A | PSMD13 | 30.63 | ANP32B | N/A |
| DNAJC11 | 75.70 | PSMD2 | N/A | CAPRIN1 | N/A |
| DPM3 | N/A | PSMD6 | N/A | LRPPRC | N/A |
| EEF1A2 | 24.53 | RPL26 | 20.81 | NFIA | 72.47 |
| EEF2 | N/A | S100A4 | N/A | PHB | N/A |
| FAM73B | N/A | SDF2L1 | 21.32 | PPP6C | 17.55 |
| FLJ20303 | N/A | SDF4 | 28.85 | SFRS10 | N/A |
| GBF1 | N/A | SF3A2 | N/A | STK38 | 123.81 |
| GEMIN4 | N/A | SF3B4 | N/A | TESC | 40.75 |
| HNRNPK | 17.88 | SFRS2B | 11.18 | JAK1 | 23.93 |
| IARS | 126.54 | SNRPC | 15.81 | PCDHB12 | 49.28 |
| IGF2BP2 | N/A | SNRPD3 | N/A | NDUFA10 | 65.44 |
| ITGA3 | 118.53 | SQSTM1 | 18.59 | RCN1 | N/A |
| ITGB4BP | N/A | TAF15 | 32.28 | | |
| ITM2B | 17.88 | TOMM40 | 44.32 | | |

### [Example 2]

In Example 1, in 299 host genes in which an influenza virus titer was reduced by a common logarithmic value of 2 or more due to siRNA introduction, it was examined whether a known inhibitor could be used as an anti-influenza virus agent.

First, using a DrugBank database (refer to Non Patent Literature 12), IPA (Ingenuity), and a database of a pharmaceutical manufacturer (Millipore, Sigma Aldrich, Selleck Chemicals), compounds serving as inhibitors of functions of these host genes were examined. As a result, 61 compounds were found as inhibitors for 44 host genes.

11 inhibitors shown in Table 11 were selected from the 61 compounds and an influence of these compounds on influenza virus replication was examined. Among them, Bortezomib and Colchicine are known as influenza virus replication inhibitors (refer to Non Patent Literatures 13 and 14).

**[Table 11]**

| Drug name | Target protein | Function |
|---|---|---|
| Bortezomib | PSMB2, PSMD2 | Proteasome inhibitor |
| 2,3-Butanedione 2-Monoxime | BAT1, DHX15, HSPD1, PSMC1, PSMC3, PSMC4, PSMC6, PSMD6, VCP | Myosin ATPase inhibitor |
| Carboxyatractyloside | SLC25A5 | A highly selective and strong inhibitor (Ki<10 nM) of an adenine nucleotide transporter (ANT) and an inducing factor of an opening of a membrane permeable transition hole (PTP). A nucleoside binding site of the ANT is stabilized on a cytoplasmic side of the intima and an exchange between ATP in mitochondria and ADP in the cytoplasm is blocked. |
| Colchicine | TUBA1, TUBB, TUBB2A | Antimitotic agent (binds to tubulins and disrupts microtubules by inhibiting its polymerization) |
| 17-Dimethylaminoethylamino -17-demethoxygeldanamycin (17-DMAG) | HSP90AB1 | HSP90 inhibitor |
| Golgicide A | GBF1 | A cell-permeable quinoline compound (reversibly reduces intracellular vesicle transportation through GBF1) selectively targeting ArfGEF and GBF1 (but not targeting BIG1/2) |
| PPIase-Parvulin Inhibitor | PPIB | Pin1/Par14 PPIase inhibitor |
| Decitabine | DNMT1 | DNMT inhibitor |
| Ruxolitinib | JAK1 | JAK1/JAK2 inhibitor |
| Pepstatin A | CTSD | Cathepsin D, pepsin, and renin inhibitor |
| WP1130 | USP9X | Deubiquitinating enzyme (DUB) inhibitor |

Specifically, HEK293 cells or A549 cells were infected with an influenza virus with an MOI of 0.001. Cells 1 hour after the infection were washed and then cultured in a culture solution containing inhibitors, a DMSO solution (final concentration: 1 volume%) was used as a control instead of the inhibitors. After culture for 48 hours in the presence of the inhibitor, a culture supernatant was collected, and a virus titer was obtained in the same manner as in Example 1. In addition, a cell viability (%) was calculated using a CellTiter-Glo assay system (commercially available from Promega Corporation) according to the appended instructions. The number of cells cultured in a medium containing the inhibitors with respect to the number of living cells in cells cultured in a medium containing the control (the DMSO solution) was calculated as the cell viability (%).

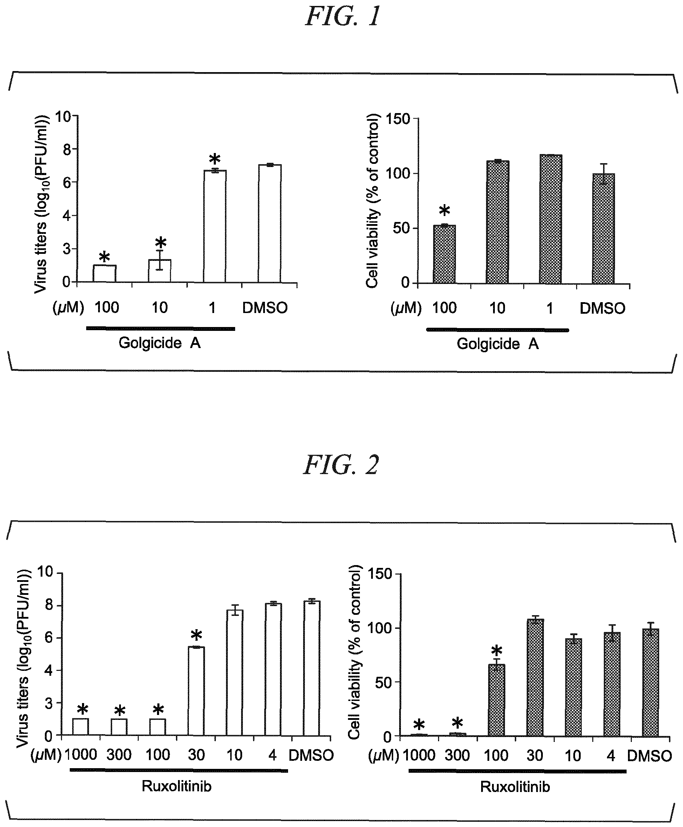

As a result, it was found that 2,3-Butanedione 2-Monoxime (30 mM), and WP1130 (50 µM) could reduce a virus titer by a common logarithmic value of 5 or more, but cell viability was significantly reduced and toxicity to host cells was strong. Bortezomib (0.2 µM) and Colchicine (2.5 µM), which are known anti-influenza virus agents, could reduce a virus titer by a common logarithmic value of 4 (Bortezomib) or 2 (Colchicine) in A549 cells without showing severe cytotoxicity. On the other hand, Golgicide A (10 µM) and Ruxolitinib (30 µM), whose relationships with influenza virus replication had not been indicated, significantly reduced a virus titer. Ruxolitinib (30 µM) did not show remarkable cytotoxicity. In Golgicide A (10 µM), a decrease in the cell viability was confirmed in HEK293 cells and was not confirmed in A549 cells. The result of Golgicide A is shown in Fig. 1. The result of Ruxolitinib is shown in Fig. 2. Based on such results, it was found that Histone acetyltransferase inhibitor II, Genistein, 2,3-Butanedione 2-Monoxime, WP1130, Golgicide A and Ruxolitinib had an anti-influenza virus effect similarly to Bortezomib and Colchicine, and among them, Golgicide A and Ruxolitinib had relatively low toxicity to host cells and were extremely useful as an anti-influenza virus agent.

### [Example 3]

Ruxolitinib is an inhibitor for JAK1 which is tyrosine kinase. As described in Example 1, a VLP production efficiency based on the M1 protein in cells in which expression of JAK1 was reduced due to siRNA introduction was 57.7% and was close to the set cutoff value of 50%. Therefore, cells into which siRNA of JAK1 was introduced were observed with an electron microscope and an influence of a decrease in expression of JAK1 genes on virus particle formation of influenza viruses was examined.

Specifically, first, in the same manner as in <siRNA>, siRNA of JAK1 genes or a control siRNA was transfected into HEK293 cells. Cells after the second transfection were infected with an influenza virus with an MOI of 5. Cell ultrathin sections were prepared from cells 12 hours after the infection according to Noda's method (Non Patent Literature 15) and observed with an electron microscope. The Tecnai (registered trademark) F20 electron microscope (commercially available from FEI) was used as the electron microscope.

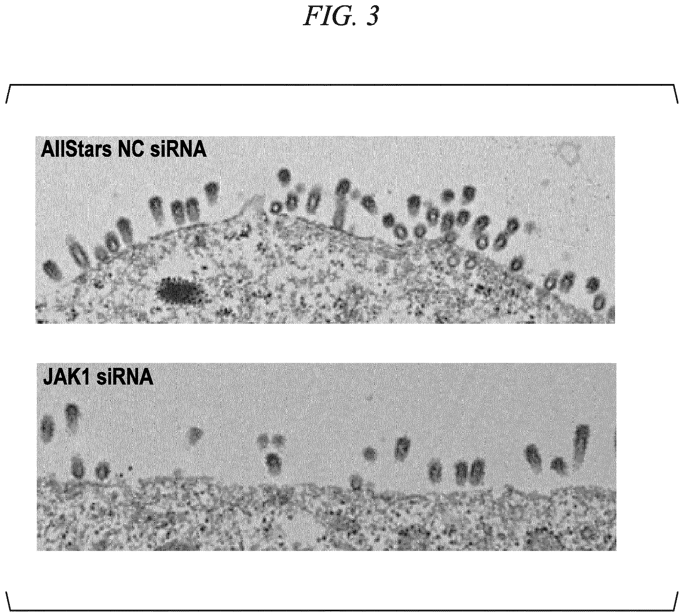

An electron micrographic image (an upper side) of cells into which the control siRNA was introduced and an electron microscope image (a lower side) of cells into which siRNA of JAK1 genes was introduced are shown in Fig. 3. In the cells into which siRNA of JAK1 genes was introduced, it was found that the formed virus-like particles were distinctly less than cells into which the control siRNA was introduced and virus particle formation was decreased by decreasing expression of JAK1 genes. The results indicate that JAK1 played an important role in a later stage of the influenza virus replication cycle.

### [Example 4]

Using protein inhibitors shown in Table 12 as test compounds, cell toxicity and an effect on influenza virus replication were examined.

**[Table 12]**

| | Inhibitor name | CAS number | Function |
|---|---|---|---|
| J1 | Tofacitinib (CP-690550) Citrate | 477600-75-2 | JAK inhibitor |
| J4 | Tyrphostin B42 | 133550-30-8 | JAK inhibitor |
| J6 | Baricitinib | 1187594-09-7 | JAK inhibitor |
| J7 | AT9283 | 896466-04-9 | JAK inhibitor |
| J8 | Momelotinib | 1056634-68-4 | JAK inhibitor |
| J11 | CEP33779 | 1257704-57-6 | JAK inhibitor |
| J13 | NVP-BSK805 2HCl | 1092499-93-8 (free base) | JAK inhibitor |
| J15 | ZM39923 HCl | 1021868-92-7 | JAK inhibitor |
| J19 | Filgotinib | 1206161-97-8 | JAK inhibitor |
| J20 | JANEX-1 | 202475-60-3 | JAK inhibitor |
| J21 | NVP-BSK805 dihydrochloride | 1092499-93-8(free base) | JAK inhibitor |
| J23 | SB1317 | 937270-47-8 | JAK inhibitor |
| J25 | WP1130 | 856243-80-6 | DUB inhibitor |

### <Cytotoxicity test>

First, as test compound solutions, test compounds were prepared in 1% dimethylsulfoxide-containing MEMs so that a final concentration was 1000, 100, 10, 1, 0.1, 0.01, or 0.001 µM.

Cell fluids in which MDCK cells, A549 cells, and HEK293 cells were prepared at concentrations of 6.25x10⁵ cells/mL, 2.5x10⁶ cells/mL, and 1.25x10⁵ cells/mL in minimum essential mediums (MEMs) were added to a 96-well plate at 0.1 mL/well and the cells were seeded. The cells in the 96-well plate were cultured in a carbon dioxide incubator at 37 °C and washed with MEM the day after the seeding date. In the washed cells in the 96-well plate, 0.1 mL of each test compound solution was added to three wells for each concentration and the cells in the 96-well plate were then cultured in a carbon dioxide incubator at 37 °C for 48 hours. After the culture, a Cell Counting Kit-8 solution (commercially available from Dojindo Molecular Technologies, Inc.) was added to each well at 10 µL and culture was additionally performed at 37 °C for several hours. After the culture, an absorbance at 450 nm of a solution of each well in the 96-well plate was measured using a microplate reader. An absorbance value when a solvent (1% dimethylsulfoxide-containing MEM) was added in place of the test compound was set to 100% at which the whole cells survived. A final concentration value of the test compound when an absorbance was 50% was calculated as a 50% cytotoxicity concentration (CC50 value).

### <Antiviral effect test>

First, as test compound solutions, test compounds were prepared in 1% dimethylsulfoxide-containing MEMs so that a final concentration was 1000, 100, 10, 1, 0.1, 0.01, or 0.001 µM.

Cell fluids in which MDCK cells, A549 cells, and HEK293 cells were prepared at concentrations of 1.25x10⁶ cells/mL, 5x10⁶ cells/mL, and 2.5x10⁵ cells/mL in MEMs were added to a 96-well plate at 0.1 mL/well and the cells were seeded. The cells in the 96-well plate were cultured in a carbon dioxide incubator at 37 °C and washed with MEM the day after the seeding date. In the washed cells in the 96-well plate, 0.1 mL of each test compound solution was added to three wells for each concentration and the cells in the 96-well plate were then cultured in a carbon dioxide incubator at 37 °C for 1 hour. After the culture, the test compound solutions were removed from the wells, and 50 µL of influenza virus A/WSN RG#1-1 strains were infected so that an MOI (the number of virus infections per cell) was 0.001 and cultured in a carbon dioxide incubator at 37 °C for 1 hour. After the culture, the virus solutions were removed from the wells, a test compound solution containing 1 µg/mL trypsin was added at 0.1 mL/well, and culture was additionally performed at 37 °C for 48 hours. It was examined whether there were viruses in the wells after the culture. A final concentration value of the test compound calculated such that there were no viruses in half (50%) of culture wells to which test compounds with the same concentration were added was calculated as a 50% virus replication inhibitory concentration (IC50). Also, determination of whether there were viruses in the culture wells was performed by determining whether aggregation occurred when 50 µL of a red blood cell solution containing 1% guinea pig erythrocytes was added to 50 µL of a culture solution collected from the culture wells.

The common logarithmic values of CC50 and IC50 of the protein inhibitors are shown in Table 13. In the table, blanks indicate that a difference between IC50 and CC50 was less than 10 times (a difference as a common logarithmic value was less than 1). As a result, in at least any of MDCK cells, A549 cells, and HEK293 cells, these protein inhibitors had IC50 whose concentration was lower than CC50 by a factor of 10 or more. That is, these protein inhibitors can suppress influenza virus replication without significantly impairing a proliferative ability of host cells and were suitable as an active ingredient of an anti-influenza virus agent.

**[Table 13]**

| | Cell lines | | | | | |
|---|---|---|---|---|---|---|
| | MDK | | A549 | | 293 | |
| | Concentration (log₁₀, nM) | | | | | |
| Compound | IC50 | CC50 | IC50 | CC50 | IC50 | CC50 |
| J1 | | | | | 4.50 | >5.5 |
| J4 | | | 4.50 | 6.43 | | |
| J6 | | | 4.50 | >5.5 | 4.50 | >5.5 |
| J7 | | | 3.50 | >5.5 | | |
| J8 | | | | | 4.50 | >5.5 |
| J11 | | | | | 3.50 | >5.5 |
| J13 | | | | | 3.17 | 4.32 |
| J15 | | | | | 2.50 | 4.68 |
| J19 | 4.50 | >5.5 | 4.50 | >5.5 | 4.50 | >5.5 |
| J20 | | | | | 4.50 | >5.5 |
| J21 | | | | | 3.50 | 4.59 |
| J23 | 1.50 | 3.90 | 1.50 | 4.55 | 1.50 | 4.72 |
| J25 | | | | | 2.50 | 3.69 |

## Claims

1. An anti-influenza virus agent that:
- suppresses expression of a JAK1 gene that encodes a protein involved in incorporation of an influenza virus vRNA or an NP protein into influenza virus-like particles in host cells, or
- suppresses the enzyme function of the JAK1 protein, for the prevention and/or the treatment of influenza virus infection.

2. The anti-influenza virus agent according to claim 1, wherein the anti-influenza virus agent is at least one selected from the group including Ruxolitinib and Tofacitinib.

3. A screening method for an anti-influenza virus agent which is a method of screening a candidate compound for an anti-influenza virus agent, wherein the candidate compound is capable of suppressing or inhibiting expression of a JAK1 gene, comprising:
- a process in which a target compound to be evaluated as a candidate compound for an anti-influenza virus agent is introduced into cells;
- a process in which an expression level of the gene in the cells into which the compound is introduced is measured; and
- a process in which, when the expression level of the gene is significantly lower than that of cells into which the compound is not yet introduced, the compound is selected as the candidate compound for the anti-influenza virus agent.

4. A screening method for an anti-influenza virus agent which is a method of screening a candidate compound for an anti-influenza virus agent, wherein the candidate compound is capable of suppressing or inhibiting expression of a JAK1 protein,
- wherein the protein that the gene encodes is an enzyme, and
- wherein the screening method includes:
a process in which enzyme activity of the protein that the gene encodes is measured under the presence of a target compound to be evaluated as a candidate compound for an anti-influenza virus agent; and
a process in which, when the enzyme activity of the protein in the presence of the compound is lower than that in the absence of the compound, the compound is selected as the candidate compound for the anti-influenza virus agent.

## Patentansprüche

1. Anti-Influenzavirus-Mittel, das:
- Expression eines JAK1-Gens unterdrückt, das für ein Protein codiert, das am Einführen einer Influenzavirus-vRNA oder eines NP-Proteins in influenzavirusähnliche Partikel in Wirtszellen beteiligt ist, oder
- die Enzymfunktion des JAK1-Proteins unterdrückt,
für die Vorbeugung und/oder die Behandlung von einer Influenzavirus-Infektion.

2. Anti-Influenzavirus-Mittel nach Anspruch 1, wobei das Anti-Influenzavirus-Mittel mindestens eines ist, ausgewählt aus der Gruppe, die Ruxolitinib und Tofacitinib einschließt.

3. Screening-Verfahren für ein Anti-Influenzavirusmittel, das ein Verfahren zum Screening einer Testverbindung für ein Anti-Influenzavirus-Mittel ist, wobei die Testverbindung geeignet ist, eine Expression eines JAK1-Gens zu unterdrücken oder zu verhindern, umfassend:
- einen Prozess, in dem eine zu bewertende Zielverbindung als eine Testverbindung für ein Anti-Influenzavirus-Mittel in Zellen eingeführt wird;
- einen Prozess, in dem ein Expressionsniveau des Gens in der Zelle, in welche die Verbindung eingeführt wird, gemessen wird; und
- einen Prozess, in dem, wenn das Expressionsniveau des Gens signifikant niedriger ist als das von Zellen, in welche die Verbindung noch nicht eingeführt wurde, die Verbindung als Testverbindung für das Anti-Influenzavirusmittel ausgewählt wird.

4. Screening-Verfahren für ein Anti-Influenzavirusmittel, das ein Verfahren zum Screening einer Testverbindung für ein Anti-Influenzavirus-Mittel ist, wobei die Testverbindung geeignet ist, eine Expression eines JAK1-Proteins zu unterdrücken oder zu verhindern,
- wobei das Protein, für das das Gen codiert, ein Enzym ist, und
- wobei das Screening-Verfahren einschließt:
einen Prozess, in welchem Enzymaktivität des Proteins, für das das Gen codiert, in der Gegenwart einer zu bewertenden Zielverbindung als eine Testverbindung für ein Anti-Influenzavirusmittel gemessen wird; und
einen Prozess, in welchem, wenn die Enzymaktivität des Proteins in der Gegenwart der Verbindung niedriger ist als die in der Abwesenheit der Verbindung, die Verbindung als die Testverbindung für das Anti-Influenzavirusmittel ausgewählt wird.

## Revendications

1. Agent contre le virus de la grippe qui :
- supprime l'expression d'un gène JAK1 qui code une protéine impliquée dans l'introduction d'un ARNv du virus de la grippe ou d'une protéine NP dans des particules pseudovirales de la grippe à l'intérieur de cellules hôtes, ou
- supprime la fonction enzymatique de la protéine JAK1, pour la prévention et/ou le traitement d'une infection par le virus de la grippe.

2. Agent contre le virus de la grippe selon la revendication 1, dans lequel l'agent contre le virus de la grippe est au moins un sélectionné dans le groupe incluant le Ruxolitinib et le Tofacitinib.

3. Procédé de criblage pour un agent contre le virus de la grippe qui est un procédé pour cribler un composé candidat pour un agent contre le virus de la grippe, dans lequel le composé candidat est capable de supprimer ou d'inhiber l'expression d'un gène JAK1, comprenant :
- un procédé dans lequel un composé cible devant être évalué en tant que composé candidat pour un agent contre le virus de la grippe est introduit dans des cellules ;
- un procédé dans lequel un niveau d'expression du gène dans les cellules dans lesquelles le composé est introduit est mesuré ; et
- un procédé dans lequel, lorsque le niveau d'expression du gène est significativement inférieur à celui des cellules dans lesquelles le composé n'est pas encore introduit, le composé est sélectionné en tant que composé candidat pour l'agent contre le virus de la grippe.

4. Procédé de criblage pour un agent contre le virus de la grippe qui est un procédé pour cribler un composé candidat pour un agent contre le virus de la grippe, dans lequel le composé candidat est capable de supprimer ou d'inhiber l'expression d'une protéine JAK1,
- dans lequel la protéine que le gène code est une enzyme, et
- dans lequel le procédé de criblage inclut :
un procédé dans lequel l'activité enzymatique de la protéine que le gène code est mesurée en présence d'un composé cible devant être évalué en tant que composé candidat pour un agent contre le virus de la grippe ; et
un procédé dans lequel, lorsque l'activité enzymatique de la protéine en présence du composé est inférieure à celle en l'absence du composé, le composé est sélectionné en tant que le composé candidat pour l'agent contre le virus de la grippe.


### SEQUENCE LISTING

<110> Japan Science and Technology Agency
   <120> Anti-influenza virus agent and Method for screening of anti-influenza virus agent
   <130> PC-20682
   <160> 5
   <170> Patent In version 3.1
<210> 1
   <211> 21
   <212> RNA
   <213> Artificial Sequence
   <220>
   <223> Description of Artificial Sequence: NP gene of WSN virus
   <400> 1
   ggaucuuauu ucuucggagu u 21
<210> 2
   <211> 45
   <212> DNA
   <213> Artificial Sequence
   <220>
   <223> Description of Artificial Sequence: vRNAtag_NP_1F Primer.
   <400> 2
   ggccgtcatg gtggcgaata gcaaaagcag ggtagataat cactc 45
<210> 3
   <211> 19
   <212> DNA
   <213> Artificial Sequence
   <220>
   <223> Description of Artificial Sequence: vRNAtag Primer.
   <400> 3
   ggccgtcatg gtggcgaat 19
<210> 4
   <211> 20
   <212> DNA
   <213> Artificial Sequence
   <220>
   <223> Description of Artificial Sequence: WSN-NP_100R Primer.
   <400> 4
   gttctccatc agtctccatc 20
<210> 5
   <211> 25
   <212> DNA
   <213> Artificial Sequence
   <220>
   <223> Description of Artificial Sequence: IDT\201CWSN-NP_46-70 Primer.
   <400> 5
   atggcgacca aaggcaccaa acgat 25